# EUROPÄISCHE PATENTSCHRIFT

(11) **EP 3 768 197 B1**
(45) Veröffentlichungstag und Bekanntmachung des Hinweises auf die Patenterteilung: **28.12.2022**
(21) Anmeldenummer: 19712164.3
(22) Anmeldetag: 15.03.2019
(51) Int. Cl.: A61F 2/78, A61F 5/00, A61F 2/50

(54) **ORTHESE, ORTHESEN- ODER PROTHESENKOMPONENTEN SOWIE VERFAHREN ZU DEREN HERSTELLUNG**
ORTHOSIS, ORTHOSIS OR PROSTHESIS COMPONENTS, AND METHOD FOR THE PRODUCTION THEREOF
ORTHÈSE, COMPOSANTS D'ORTHÈSE OU DE PROTHÈSE ET PROCÉDÉ DE FABRICATION ASSOCIÉ

(30) Priorität: 20.03.2018 DE 102018106573
(43) Veröffentlichungstag der Anmeldung: 27.01.2021
(73) Patentinhaber: Otto Bock Healthcare Products GmbH, 1110 Wien (AT)
(72) Erfinder: SIEWERT, Gordon, 37083 Göttingen (DE); OVERDEVEST, Etienne, 37073 Göttingen (DE); VOLKMAR, Marco, 37115 Duderstadt (DE)
(74) Vertreter: Gramm, Lins & Partner Patent- und Rechtsanwälte PartGmbB
(86) Internationale Anmeldenummer: PCT/EP2019/056581
(87) Internationale Veröffentlichungsnummer: WO 2019/179894

(56) Entgegenhaltungen:
- US-A- 5 288 287
- US-B2- 7 708 709
- BARTKUS E K ET AL: "COMPOSITE PROSTHESIS JOINS PERFORMANCE, ECONOMY, COMFORT", MODERN PLASTICS INTERNATIONAL, MCGRAW-HILL,INC. LAUSANNE, CH, Bd. 23, Nr. 7, 1. Juli 1993 (1993-07-01), Seiten 39-41, XP000383439, ISSN: 0026-8283

## Beschreibung

Die Erfindung betrifft ein Verfahren zum Herstellen von Orthesen- oder Prothesenkomponenten zur Aufnahme von oder zur Befestigung an einem Körperteil sowie Orthesen- und Prothesenkomponenten und eine Orthese aus mehreren Orthesenkomponenten, wie in den Ansprüchen definiert.

Orthesen- oder Prothesenkomponenten zur Aufnahme von oder zur Befestigung an einem Körperteil sind insbesondere Prothesenschäfte, in die ein Stumpf einer Gliedmaße eingeführt wird, oder Orthesenschalen oder Spangen, die an den Körper angelegt und daran befestigt werden, um gelenkübergreifend mit einer zweiten Orthesenkomponente über eine Gelenkeinrichtung verbunden zu werden. Prothesenschäfte werden häufig aus faserverstärkten Kunststoffen hergestellt, die auf Träger aufgelegt, mit Harz getränkt und anschließend ausgehärtet werden. Die Träger können als Standardvorlagen ausgebildet oder auf Basis eines Abgusses des jeweiligen Stumpfes erstellt werden. An einem distalen Ende des Prothesenschaftes wird eine Ankerplatte zur Festlegung eines Pyramidenadapters oder einer Adapteraufnahme eingegossen oder befestigt, so dass der Prothesenschaft mit einer distalen Gelenkeinrichtung und distalen Prothesenkomponenten verbunden werden kann.

Orthesenkomponenten zur Aufnahme von Körperteilen oder zur Befestigung an Körperteilen können aus Kunststoffkomponenten hergestellt sein. Ebenfalls können diese Komponenten, die als Schalen oder Spangen oder dergleichen ausgebildet sein können, aus faserverstärkten Kunststoffmaterialien hergestellt sein. Häufig sind die Orthesenkomponenten über Befestigungseinrichtungen wie Gurte oder Schnallen an dem Körperteil festlegbar. Über die Befestigungseinrichtungen wird die Gliedmaße umschlossen, gegebenenfalls werden die Orthesenkomponenten elastisch verformt. Dementsprechend sind die Orthesenkomponenten in einem begrenzten Maße elastisch verformbar.

Zur Herstellung von Orthesen mit Orthesenkomponenten zur Anlage und Aufnahme von Körperteilen oder Gliedmaßen werden bei individuell angefertigten Orthesen Gelenkeinrichtungen zusammen mit den Orthesenkomponenten auf einem Modell der Gliedmaße befestigt und einlaminiert. Alternativ werden Befestigungselemente für Gelenkeinrichtungen über Fixiereinrichtungen, sogenannte Dummies oder Platzhalter zueinander orientiert gehalten, die bei der Aushärtung und Fertigstellung der Orthesenkomponenten an Ort und Stelle verbleiben müssen.

Der Artikel "COMPOSITE PROSTHESIS JOINS PERFORMANCE, ECONOMY, COMFORT"; MODERN PLASTICS INTERNATIONAL, 19930701 MCGRAW-HILL, INC. LAUSANNE, CH - ISSN 0026-8283, Vol:23, Nr:7, Page(s):39 - 41 von E. K. Bartkus et al beschreibt die Herstellung von Prothesen und Prothesenkomponenten aus Faserverbundwerkstoffen.

Die US 7 708 709 B2 betrifft eine Orthese mit einer Grundeinheit mit einem Abstützteil, das zur Anlage an einem Unterarm ausgebildet ist, das Abstützteil weist ein Abstützpolster auf. Eine Mittelhandeinheit zur Anlage an dem Handrücken ist mit einem Polster versehen und gelenkig mit der Grundeinheit verbunden. Die gelenkige Verbindung erfolgt über ein Schwenkgelenksystem, das eine Schale aufweist, die sich zwischen der Grundeinheit und der Mittelhandeinheit erstreckt. Vertikale Schwenkverbinder ermöglichen eine Verschwenkung um eine erste Achse, seitliche Schwenkverbinder ermöglichen eine Verschwenkung um eine senkrecht dazu orientierte Achse, wenn die Orthese angelegt ist. Endanschläge begrenzen die Beweglichkeit des Handgelenks. Die Grundeinheit, die Mittelhandeinheit und die Schale können aus faserverstärktem Kunststoff gebildet sein. Die Polster sind auf der dem Arm oder der Hand zugewandten Seite angeordnet.

Die US 5 288 287 A betrifft eine Knieorthese mit einem Oberschenkelrahmen und einem Unterschenkelrahmen, die aus einem Faserverbundwerkstoff hergestellt sind. Auf der Innenseite sind Polsterelemente angeordnet. Über Gurte, die an Schlaufen befestigt sind, können die gelenkig aneinander befestigten Rahmenteile an dem Bein festgelegt werden.

Aufgabe der vorliegenden Erfindung ist es, ein Verfahren zum Herstellen von Orthesen- oder Prothesenkomponenten zur Aufnahme eines Körperteils oder zur Befestigung an einem Körperteil bereitzustellen, das einfacher und kostengünstiger durchzuführen ist. Ziel ist eine einfache und sichere Befestigung von Gelenkkomponenten und Funktionskomponenten sowie deren anforderungsgerechte Befestigung.

Erfindungsgemäß wird diese Aufgabe durch ein Verfahren mit den Merkmalen des Hauptanspruches sowie durch die Orthesen- oder Prothesenkomponente oder Orthese aus mehreren solcher Orthesenkomponenten gemäß der nebengeordneten Ansprüche gelöst. Vorteilhafte Ausgestaltungen und Weiterbildungen der Erfindung sind in den Unteransprüchen, der Beschreibung sowie den Figuren offenbart.

Das erfindungsgemäße Verfahren zur Herstellung von Orthesenkomponenten oder Prothesenkomponenten zur Aufnahme eines Körperteils oder zur Befestigung an einem Körperteil sieht zunächst das Aufbringen einer Basislage auf einen zu der Körperteilform korrespondierenden Träger vor. Der Träger kann als ein Abguss des Körperteils, ein modifizierter Abguss des Körperteils, eine auf eine andere Art und Weise hergestellte Form des Körperteils oder eines modifizierten Körperteils ausgebildet oder das Körperteil selbst sein. Die Modifizierung kann bei einem Abguss durch Entfernen oder Hinzufügen von Material oder Auflegen einer Polsterschicht erfolgen, bei einem auf andere Art und Weise hergestellten Träger durch Modifizierung des Datensatzes, so dass beispielsweise nach einer optischen oder anderweitigen Erfassung der äußeren Form des Körperteils oder des Stumpfes der Datensatz verändert, z.B. geglättet verändert wird, so dass beim Herstellen des gegenständlichen Trägers, beispielsweise durch Ausfräsen aus einem Rohling oder durch Urformen, beispielsweise im 3D-Druckverfahren, der entsprechende Träger hergestellt wird. Die Basislage kann zunächst biegeschlaff und aushärtend ausgebildet sein. Alternativ ist es vorgesehen, dass die Basislage eine so große Eigenstabilität aufweist, dass sie nach dem Anformen auf dem Träger oder an den Träger, beispielsweise den Abguss oder nach dem Auflegen auf das Körperteil als solches, die Form nicht oder nicht wesentlich verändert, insbesondere wenn die Basislage im weiteren Handhabungsverfahren entsprechend vorsichtig gehandhabt wird. Bevor Befestigungselemente auf die Basislage aufgesetzt werden, muss die Basislage eine ausreichende Eigenstabilität aufweisen, die durch Aushärten, ggf. unter erhöhten Temperaturen erzielt werden kann.

Anschließend werden mehrere Befestigungselemente auf der Basislage in den definierten Positionen zueinander angeordnet, wobei die Befestigungselemente eine Basis aufweisen, von der zumindest ein Formschlusselement absteht. Über das Formschlusselement ist es möglich, weitere Komponenten einer Prothese oder Orthese an der Prothesenkomponente oder Orthesenkomponente festzulegen, beispielsweise einen Aktuator oder eine Gelenkeinrichtung. Unter einem Formschlusselement wird auch eine Schraube oder eine Gewindestange verstanden. Durch das Anordnen der Befestigungselemente in vorab definierten Positionen zueinander ist es möglich, aus einem Pool von Komponenten mit standardisierten Anschlusseinrichtungen auszuwählen, um diese dann an der jeweiligen Orthesenkomponente oder Prothesenkomponente festzulegen. Damit ist beispielsweise möglich, Orthesenkomponenten individuell an den jeweiligen Patienten oder Orthesennutzer anzupassen und unterschiedliche Aktuatoren oder Gelenkeinrichtungen oder auch Korrektureinrichtungen an den Komponenten anzuordnen, die sich beispielsweise an einen Genesungsfortschritt anpassen oder über die auf veränderte Umstände, beispielsweise Verschlimmerungen bei Erkrankungen reagiert werden kann. Die Basis des jeweiligen Befestigungselementes, insbesondere aller Befestigungselemente, die auf der Basislage angeordnet werden, liegt entweder auf der Basislage auf oder ist zumindest der Basislage zugewandt. Die Basis stellt bevorzugt eine gegenüber dem Formschlusselement vergrößerte Oberfläche zur Verfügung, um eine bessere Zuordnung und gegebenenfalls Krafteinleitung zu gewährleisten. Die der Basislage zugewandte Oberfläche der Basis kann direkt auf der Basislage aufliegen oder über ein Zwischenteil, beispielsweise ein Abstandselement oder eine formbare, vorzugsweise aushärtende Masse der Basislage zugeordnet werden. Nach dem Anordnen der Befestigungselemente wird zumindest eine Lage eines Faserverbundwerkstoffes, beispielsweise eines Prepregs, auf die Basislage gelegt und die Basis eingebettet, wobei das Formschlusselement von der der Basislage abgewandten Seite aus zugänglich bleibt. Durch die Zugänglichkeit des Formschlusselementes von der der Basislage abgewandten Seite ist es möglich, nach der Fertigstellung der Orthesenkomponente oder Prothesenkomponente eine weitere Komponente an dem Befestigungselement festzulegen. Anschließend wird die zumindest eine Lage des Faserverbundwerkstoffes ausgehärtet und somit die Orthesenkomponente oder Prothesenkomponente fertiggestellt.

Eine Weiterbildung des Verfahrens sieht vor, dass die Basislage mit der zumindest einen Lage des Faserverbundwerkstoffes verbunden wird und die Basis des Befestigungselementes zwischen der Basislage und der zumindest einen Lage des Faserverbundwerkstoffes einlaminiert wird. Durch die Verbindung oder das Auflegen der Basislage mit oder auf der Faserverbundwerkstofflage wird die formstabile Orthesenkomponente oder Prothesenkomponente mit einer auf der Innenseite der Basis des Befestigungselementes angeordneten Lage zumindest teilweise abgedeckt. Dadurch wird verhindert, dass das Befestigungselement von der Faserverbundwerkstofflage getrennt werden kann. Die Basislage selbst kann auch als Faserverbundwerkstofflage, beispielsweise aus einem Prepreg, ausgebildet sein, so dass sich eine dauerhafte und starre Verbindung mit einer formschlüssigen Festlegung des Befestigungselementes auf der Innenseite der aufgelegten Faserverbundwerkstofflage ergibt. Die Basislage kann nach Fertigstellung der Orthese oder Orthesenkomponente von der zumindest einen Faserverbundwerkstofflage entfernt werden.

In einer Weiterbildung des Verfahrens ist das Formschlusselement an oder in einem Schaft ausgebildet oder angeordnet, wobei der Schaft teilweise in die zumindest eine Faserverbundwerkstofflage eingebettet wird und teilweise aus der Faserverbundwerkstofflage herausragt. Der Schaft steht von der Basis des Befestigungselementes ab, wobei die Basis einen größeren Querschnitt als der Schaft aufweist, so dass sich ein Absatz ausbildet, auf dem die aufgelegte Lage des Faserverbundwerkstoffes aufliegt. Durch das teilweise Herausragen des Schaftes ist es möglich, das Formschlusselement leicht zugänglich zu halten, um die an die Orthesenkomponente oder Prothesenkomponente anzubringenden Ortheseneinrichtungen oder Protheseneinrichtungen festlegen zu können.

Die Schäfte mehrerer Befestigungselemente können parallel zueinander ausgerichtet sein. Insbesondere können Schäfte einer Gruppe von Befestigungselementen, die an einer besonderen Ortheseneinrichtung angeordnet sind, parallel zueinander ausgerichtet werden. Eine besondere Orthesenkomponente ist beispielsweise eine Orthesenkomponente, die proximal oder distal einer Gelenkachse einer Gelenkeinrichtung angeordnet ist, beispielsweise eine Oberschenkelschale oder Oberschenkelspange und eine Unterschenkelschale oder Unterschenkelspange. Wenn die Schäfte aller Befestigungselemente parallel zueinander ausgerichtet werden, erleichtert dies die Montage der daran befestigten oder zu befestigenden Elemente wie Gelenkeinrichtung oder Aktuator. Die parallel zueinander ausgerichteten Schäfte einer Gruppe können gegenüber der parallelen Ausrichtung der Schäfte einer anderen Gruppe verkippt angeordnet sein.

In einer weiteren Weiterbildung der Erfindung ist es vorgesehen, dass an dem Befestigungselement zumindest eine Anbindungsfläche angeordnet oder ausgebildet ist, die von der Basis beabstandet ist und nicht von der zumindest einen Faserverbundwerkstofflage abgedeckt ist. Die Anbindungsfläche stellt eine definierte Fläche zur Anordnung einer weiteren Einrichtung, beispielsweise eines Dämpfers oder einer Gelenkeinrichtung, bereit und ermöglicht dessen präzise Zuordnung und feste Verbindung über das Formschlusselement. Wenn alle Anbindungsflächen aller Befestigungselemente auf einer Orthesenkomponente oder Prothesenkomponente frei zugänglich sind, ist es vereinfacht möglich, weitere Komponenten an der Orthesenkomponente festzulegen, wenn deren korrespondierenden Kontaktflächen oder Anbindungsflächen ebenfalls in einer Ebene liegen oder zumindest parallel zueinander ausgerichtet sind. Bevorzugt liegen alle Anbindungsflächen in einer Ebene oder sind zumindest parallel zueinander in parallelen Ebenen ausgerichtet, wodurch eine präzise Ausrichtung und Orientierung der an der Orthesenkomponente oder Prothesenkomponente festzulegenden Einrichtung möglich ist.

Die Basis des Befestigungselementes kann auf der Basislage fixiert werden, beispielsweise aufgeklebt werden oder mit einer Spachtelmasse an der Basislage fixiert werden. Über eine Klebstoffschicht, die in der Regel vergleichsweise dünn ist, wird kein oder nahezu kein Höhenausgleich erreicht. Über eine Spachtelmasse wird in der Regel neben einer dauerhaften Fixierung der Basis des Befestigungselementes und damit des Befestigungselementes selbst auf der Basislage ein Höhenausgleich erreicht, um eine Zuordnung aller Befestigungselemente in einer zueinander definierten Ebene oder zumindest in einem ebenen Bereich zueinander zu ermöglichen. Bevorzugt sind alle Befestigungselemente hinsichtlich einer Referenzebene parallel zueinander orientiert, zumindest soweit es die Befestigungselemente auf einer Orthesenkomponente an einem einzigen Gliedmaßenteil betrifft. Als ein Gliedmaßenteil wird ein Teil einer Gliedmaße angesehen, der über ein Gelenk mit einem anderen Gliedmaßenteil oder mit einem anderen Körperteil verbunden ist.

In einer Weiterbildung der Erfindung ist vorgesehen, dass der Träger als ein Positivmodell des Körperteils ausgebildet wird und das Aushärten der zumindest einen Faserverbundwerkstofflage, gegebenenfalls in Verbindung mit dem Verbinden der Basislage mit der zumindest einen Faserverbundwerkstofflage an dem Träger erfolgt. Aufgrund der Anordnung und gegebenenfalls Befestigung und Fixierung der Befestigungselemente auf der Basislage ist es möglich, die Orthesenkomponente oder Prothesenkomponente fertig vorzuformen und ohne eine zusätzliche Fixiereinrichtung oder einem externen Fixierelement zueinander ausgerichtet zu halten und das Aushärten und Stabilisieren der Prothesenkomponente oder Orthesenkomponente zu erreichen. Sofern die Basislage ausreichend stabil ist, kann das Aushärten der zumindest einen Faserverbundwerkstofflage auch ohne den Träger erfolgen. Zur endgültigen Fertigstellung der Orthesenkomponente können weitere Elemente oder Komponenten an der Orthesenkomponente oder Prothesenkomponente angeordnet werden, beispielsweise Polstereinrichtungen oder dergleichen.

Eine Weiterbildung der Erfindung sieht vor, dass in der zumindest einen aufgelegten Faserverbundwerkstofflage Ausnehmungen ausgebildet sind, durch die Teile der Befestigungselemente hindurchgeführt werden oder wird, beispielsweise der Schaft, der von der Basis abragt oder auch von der Basis abragende Formschlusselemente wie Vorsprünge, Haken oder dergleichen, über die eine formschlüssige Verriegelung mit der zumindest einen Faserverbundwerkstoff lage und somit eine zusätzliche Festlegung des Befestigungselementes an der zumindest einfachen Verbundwerkstofflage erreicht wird. Über diese zusätzlichen Ausformungen oder Anordnungen von Vorsprüngen an der Basis des Befestigungselementes kann eine Verdrehsicherung des Befestigungselementes an oder in der Faserverbundwerkstofflage oder Orthesenkomponente bzw. Prothesenkomponente erreicht werden.

Eine Weiterbildung der Erfindung sieht vor, dass die Befestigungselemente oder das zumindest eine Befestigungselement verdrehgesichert und unlösbar an der Orthesenkomponente oder Prothesenkomponente festgelegt werden. Dies kann durch die Formgebung der Basis und/oder des Schaftes erreicht werden, die oder der unrund, eckig oder mit Vorsprüngen oder abragenden Elementen ausgestattet sein kann oder können. Die Unlösbarkeit wird insbesondere durch ein Einbetten der Basis und des Formschlusselementes oder des Schaftes in die zumindest eine Faserverbundwerkstofflage erreicht. In der Basis und/oder in dem Formschlusselement und/oder dem Schaft können Vorsprünge oder Ausnehmungen ausgebildet sein, die in formschlüssigem Eingriff mit Teilen der Faserverbundwerkstofflage treten, so dass eine Verdrehung und/oder Verschiebung und Axialverlagerung in Schaftlängserstreckung verhindert wird.

Die Befestigungselemente können auf der dem Körperteil zugewandten Seite der Orthesenkomponente oder Prothesenkomponente vollständig mit der Basislage abgedeckt werden, so dass auf der Innenseite, also auf der dem Nutzer der Prothesenkomponente oder Orthesenkomponente zugewandten Seite, eine geschlossene Oberfläche vorhanden ist, was den Tragekomfort erhöht. Darüber hinaus wird verhindert, dass das Befestigungselement in Richtung auf den Orthesennutzer oder Prothesennutzer aus der Orthesenkomponente oder Prothesenkomponente hinausgedrückt wird.

Eine Weiterbildung der Erfindung sieht vor, dass Anbindungsflächen aller Befestigungselemente in einer gemeinsamen Ebene oder in einem Bereich zwischen zwei parallelen Ebenen vorpositioniert werden, wobei die Anbindungsflächen an den Befestigungselementen beabstandet zu der Basis positioniert sind. Die Ausrichtung entlang paralleler Eben muss nicht für Gruppen jenseits der Gelenkachse gegeben sein. Ein gegebenenfalls vorhandener Abstand zwischen den Basen der Befestigungselemente zu der Basislage wird über ein Ausgleichselement oder über eine Ausgleichsmasse aufgefüllt, um mit vordefinierten Befestigungselementen arbeiten zu können. Es wird somit eine Befestigungsebene für die Anbindungsflächen vorgegeben oder zumindest ein Bereich zwischen zwei Ebenen vorgegeben, innerhalb dessen sich die Anbindungsflächen der Befestigungselemente befinden müssen. Die vorhandene Distanz zwischen den Basen der standardisierten, vordefinierten Befestigungselementen und der Basislage, die individuell an den jeweiligen Nutzer der Orthesenkomponente oder Prothesenkomponente ausgebildet ist, wird dann über ein Ausgleichselement oder eine Ausgleichsmasse ausgeglichen. Das Ausgleichselement kann gleichzeitig zur Fixierung auf der Basislage dienen, gleiches gilt für die Ausgleichsmasse, die beispielweise als Spachtelmasse oder als aushärtbarer Kleber ausgebildet ist, der eine ausreichende Festigkeit und Stabilität aufweist, um die Befestigungselemente an oder in der Orthesenkomponente oder Prothesenkomponente zu halten.

Eine Weiterbildung des Verfahrens sieht vor, dass die Befestigungselemente in vorbestimmten Positionen zueinander und zu einer Gelenkachse eines Gelenks des Körperteils vorpositioniert und auf der Basislage angeordnet oder dieser zumindest zugeordnet werden. Sofern der direkte Kontakt der jeweiligen Basis mit der Basislage möglich ist, wird die Positionierung und gegebenenfalls Fixierung ohne Zwischenschaltung eines Ausgleichselementes oder einer Ausgleichsmasse vorgenommen, sofern dies nicht möglich ist, wird über ein Ausgleichselement oder eine Ausgleichsmasse der Abstand zwischen der Basis und der Basislage ausgeglichen. Die Fixierung erfolgt entweder über die Spachtelmasse oder über einen Kleber, der keine oder eine vernachlässigbare Verbindungssicht zwischen der Basis und der Basislage ausbildet. Die Positionen der Befestigungselemente zueinander und insbesondere auch die Positionen von vorhandenen Anbindungsflächen aller Befestigungselemente auf einer gemeinsamen Orthesenkomponente sind vordefiniert und entsprechen den Befestigungseinrichtungen oder Anbindungspunkten von Komponenten, die an der Orthesenkomponente oder Prothesenkomponente angeordnet werden oder angeordnet werden sollen.

Die Befestigungselemente können an einem Halter oder einer Positioniereinrichtung angeordnet und zueinander vorpositioniert werden, bevor sie auf der Basislage angeordnet oder dieser zugeordnet werden. Nach dem Anordnen der Befestigungselemente auf der Basislage oder der Zuordnung auf der Basislage und Fixierung der Befestigungselemente an der Basislage wird der Halter oder die Positioniereinrichtung entfernt

Eine Weiterbildung des Verfahrens sieht vor, dass zur Ausbildung einer Orthese mit einer Distalkomponente und einer Proximalkomponente die zumindest eine Faserverbundwerkstofflage, die auf die Basislage aufgelegt und die Basis einbettet, in einem Bereich auf dem Träger aufgelegt wird, der dem eines natürlichen Gelenkes entspricht und anschließend zur Ausbildung eines Grundkörpers ausgehärtet wird. Dabei sind Befestigungselemente vor dem Aushärten proximal und distal einer Gelenkachse des natürlichen Gelenkes angeordnet und werden in der zumindest einen Faserverbundwerkstoff lage eingebettet. Nach dem Aushärten wird die zumindest eine Faserverbundwerkstofflage des Grundkörpers und gegebenenfalls auch die Basislage, sofern diese mit der aufgelegten Faserverbundwerkstofflage gemeinsam ausgehärtet wird, in dem Bereich der Gelenkachse durchtrennt, um aus dem Grundkörper die Distalkomponente und Proximalkomponente der Orthese auszubilden. Wird keine Faserverbundwerkstofflage in dem Bereich der Gellenkachse auf die Basislage aufgelegt, kann ebenfalls ein Orthesengrundkörper mit daran festgelegten Befestigungselementen entstehen, wobei der Bereich um die Gelenkachse nur von der Basislage gebildet wird. Zum Trennen und Aufteilen in eine Proximal- und eine Distalkomponente muss dann nur die Basislage durchtrennt werden. Statt der Ausgestaltung und Ausbildung zweier Orthesenkomponenten auf einem Träger zur Anfertigung einer individuellen Orthese wird ein gelenkübergreifender Orthesengrundkörper hergestellt, der ausgehärtet wird. In dem Orthesengrundkörper sind die Befestigungselemente zur außenseitigen Anbringung einer Gelenkeinrichtung oder eines Aktuators festgelegt, wobei die Festlegung der Befestigungselemente an vorbestimmten, standardisierten Positionen in vorbestimmten Orientierungen zueinander erfolgt. Sowohl die Orientierung der Befestigungselemente oder des Befestigungselementes an der proximalen Komponente als auch die Orientierung der Befestigungselemente oder des Befestigungselementes an der distalen Orthesenkomponente sind vorbestimmt und bewegen sich in einem vorab festgelegten Toleranzbereich.

Bevorzugt sind Anbindungsflächen an den Befestigungselementen definiert, die in der jeweiligen Orthesenkomponente, also in einer proximalen Orthesenkomponente und in einer distalen Prothesenkomponente parallel zueinander und noch weiter bevorzugt in einer gemeinsamen Ebene orientiert sind. Die Ausrichtung der Anbindungsflächen an unterschiedlichen Komponenten wird ebenfalls vorab definiert und liegt in einem vorgegebenen Toleranzbereich, um eine erleichterte Zuordnung der weiteren Komponenten, insbesondere der Gelenkeinrichtung und eines Aktuators zu erleichtern.

Alle Anbindungsflächen einer Komponente sind zu der Orientierung der Ebenen der Anbindungsflächen der anderen Komponente bevorzugt um einen gleichen Winkel verkippt, wenn eine Verkippung notwendig ist, beispielsweise aufgrund der besonderen Formgebung der Gliedmaße, an der die gelenkübergreifende Orthese angelegt werden soll. Nach der Anordnung in definierten Positionen zueinander und in einer definierten Winkelorientierung innerhalb eines gewissen Toleranzbereiches zueinander wird dann im Bereich der Gelenkachse des natürlichen Gelenkes des gelenkübergreifend ausgebildeten Grundkörpers durch ein Trennverfahren der Grundkörper in eine Proximalkomponente und in eine Distalkomponente getrennt, um danach eine Gelenkeinrichtung an der proximalen Komponente und der distalen Komponente über die Befestigungselemente festzulegen und die Orthese fertigzustellen. Selbstverständlich kann nach dem Aushärten und dem Trennen noch eine weitere Bearbeitung der Orthesenkomponenten erfolgen, beispielsweise eine Glättung der Form, ein Entgraten der Ränder sowie eine Versiegelung, Lackierung und Polsterung sowie die Befestigung von Befestigungseinrichtungen wie Gurten oder dergleichen.

Zum Fertigstellen der Orthese mit den wie oben beschrieben hergestellten Orthesenkomponenten wird eine Gelenkeinrichtung an den Befestigungselementen über die Formschlusselemente festgelegt, beispielsweise verschraubt, wenn die Formschlusselemente ein Gewinde aufweisen.

Die erfindungsgemäße Orthesen- oder Prothesenkomponente zur Aufnahme eines Körperteils oder zur Befestigung an einem Körperteil weist eine Basislage auf, die auf einem zu der Körperform korrespondierend geformten Träger anordenbar ist, wobei der Träger sowohl das Körperteil selbst als auch eine Abformung oder ein Abbild des Körperteils sein kann. Auf der Basislage ist zumindest ein Befestigungselement mit einer Basis und einem von der Basis abstehenden Formschlusselement angeordnet, wobei die Basis auf der Basislage aufliegt und ihr zugewandt ist. Das Befestigungselement ist mit zumindest einer Lage, bevorzugt mehreren Lagen eines Faserverbundwerkstoffes eingebettet, und das Formschlusselement bleibt von der der Basislage abgewandten Seite aus nach dem Aushärten der Faserverbundwerkstofflage oder Faserverbundwerkstofflagen zugänglich.

Die Erfindung sieht vor, dass mehrere Befestigungselemente eingebettet sind, die einen Schaft als Formschlusselement oder mit einem Formschlusselement und zumindest eine nicht eingebettete Anbindungsfläche aufweisen, wobei die Schäfte aller Befestigungselemente einer Orthesenkomponente oder einer Prothesenkomponente parallel zueinander ausgerichtet sind. Durch die Anordnung der Schäfte parallel zueinander ist es möglich, die Montage weiterer Komponenten an dem Befestigungselement zu vereinfachen. Bevorzugt sind die Befestigungselemente in Gruppen angeordnet, beispielsweise eine Gruppe für die Proximalkomponente und eine Gruppe für die Distalkomponente. Die Orientierung der Befestigungselemente in einer Gruppe ist vorzugsweise einheitlich.

Bevorzugt sind Anbindungsflächen ebenfalls parallel zueinander ausgerichtet, so dass korrespondierende Anbindungsflächen an Komponenten, die an den Orthesenkomponenten oder Prothesenkomponenten festgelegt sind, leicht zueinander orientierbar und leicht auswechselbar daran festlegbar sind. Es können alle Anbindungsflächen parallel oder sogar in einer gemeinsamen Ebene angeordnet sein, ebenso können die Anbindungsflächen einzelner Gruppen parallel oder in einer gemeinsamen Ebene angeordnet sein. Bevorzugt liegen alle Anbindungsflächen einer Orthesenkomponente oder Prothesenkomponente in einer gemeinsamen Ebene oder zumindest zwischen zwei parallel zueinander ausgerichteten Ebenen, die versetzt zueinander angeordnet sind. Die gemeinsamen Ebenen können auch zueinander verkippt und ein einem Winkel abweichend 180° zueinander orientiert sein.

Die Basislage kann ebenfalls als eine Faserverbundwerkstofflage ausgebildet sein und eine geschlossene Oberfläche auf der dem Körperteil zugewandten Seite der Orthesenkomponente oder Prothesenkomponente ausbilden.

Das Befestigungselement kann verdrehgesichert oder unlösbar in der zumindest einen Faserverbundwerkstofflage eingebettet sein.

Das Befestigungselement kann eine unrunde Basis und/oder Vorsprünge und/oder Ausnehmungen zur Verdrehsicherung aufweisen, die formschlüssig in die zumindest eine Faserverbundwerkstofflage eingebettet und daran gehalten sind.

An dem Formschlusselement oder an dem Schaft kann ein Gewinde angeordnet und ausgebildet sein. Das Gewinde kann entweder als Innengewinde oder Außengewinde ausgebildet sein, so dass das Befestigungselement als Ankerschraube oder Ankermutter wirkt.

Befestigungseinrichtungen zur Festlegung der Orthesenkomponente oder Prothesenkomponente an einem Körperteil können an der Orthesenkomponente oder Prothesenkomponente angeordnet sein, beispielsweise Gurte, Schnallen oder auch Prothesenliner zur Ausgestaltung einer Saugschafttechnologie.

Eine Orthese aus mehreren Orthesenkomponenten, wie sie oben beschrieben worden sind, weist zumindest eine an mehreren Befestigungselementen festgelegte Gelenkeinrichtung auf. Ebenfalls kann ein Aktuator über Befestigungselemente an einer proximalen und an einer distalen Orthesenkomponente festgelegt sein.

Eine Weiterbildung der Orthese sieht vor, dass die Orthesenkomponenten miteinander ein natürliches Gelenk übergreifend verbunden sind und eine Solltrennstelle oder ein Solltrennbereich aufweisen, in der oder in dem die Gelenkachse des natürlichen Gelenkes liegt.

Nachfolgend wird ein Ausführungsbeispiel der Erfindung anhand der beigefügten Figuren näher erläutert. Es zeigen:
- Figuren 1a bis 1d: Ansichten eines Befestigungselementes;
- Figur 2: eine schematische perspektivische Ansicht eines Trägers mit einer aufgelegten Basislage;
- Figur 3: eine Basislage gemäß Figur 2 mit aufgesetzten Befestigungselementen;
- Figur 4: eine schematische Seitenansicht mit aufgelegter Faserverbundwerkstofflage;
- Figur 5: eine Schnittansicht gemäß Figur 2;
- Figur 6: eine Schnittansicht gemäß Figur 3;
- Figur 7: eine Schnittansicht gemäß Figur 4;
- Figur 8: eine Darstellung einer fertiggestellten Orthese;
- Figur 9: eine Positioniereinrichtung für die Befestigungselemente;
- Figur 10: die Positioniereinrichtung gemäß Figur 9 in Untenansicht;
- Figur 11: eine Explosionsdarstellung der Positioniereinrichtung;
- Figuren 12 und 13: Darstellungen von Haltern in unterschiedlichen Winkelstellungen,
- Figuren 14 und 15: Schnittdarstellungen der Figuren 12 und 13; sowie
- Figur 16: eine Variante des Zentralstückes.

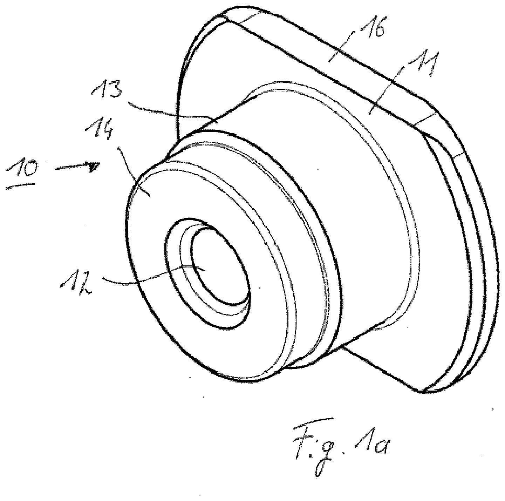

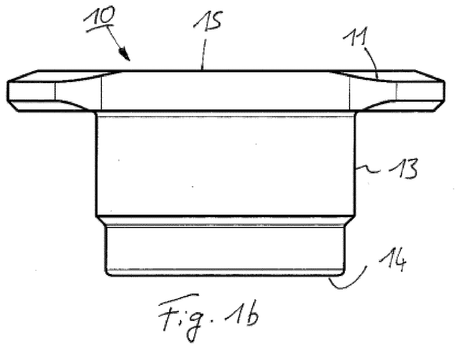

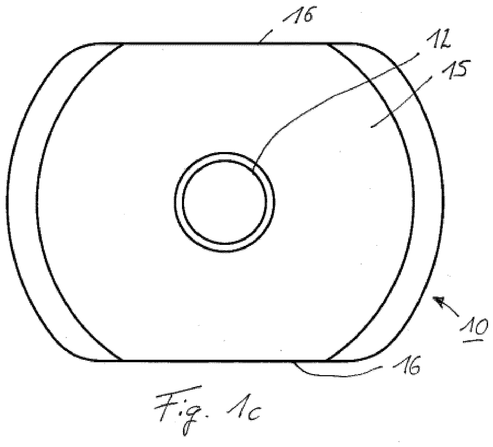

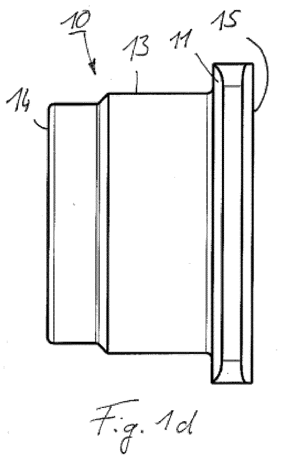

Figuren 1a bis 1d zeigen unterschiedliche Ansichten eines Befestigungselementes 10, wobei die Figur 1a eine perspektivische Gesamtansicht, die Figur 1b eine Seitenansicht, Figur 1c eine Untenansicht sowie Figur 1d eine weitere Seitenansicht sind. Das Befestigungselement 10 weist eine Basis 11 auf, die im dargestellten Ausführungsbeispiel im Wesentlichen flach und plattenförmig ausgebildet ist. An den Rändern der Basis 11 sind Abschrägungen ausgebildet, um eine verbesserte Anlage an eine Unterlage oder eine Auflage zur Ausbildung eines glatten Überganges zu bieten. Darüber hinaus kann zwischen den Abschrägungen und Faserverbindwerkstoffen Verbindungsmaterial oder ein Klebstoff angeordnet werden, um das Befestigungselement 10 daran zu fixieren. Die Basis 11 ist unrund ausgebildet und weist zwei Abflachungen 16 an einander gegenüberliegenden Seiten auf. Zwischen den Abflachungen 16 bildet die Basis 11 einen Radius aus, dessen Fortführung zu einer Kreisform führen würde. Die Kontur der Basis 11 entspricht also einem Kreis mit abgeschnittenen Kreissegmenten mit parallelen Kreissehnen. In der Mitte der Basis 11 ist eine Zentralbohrung mit einem Formschlusselement 12 in Gestalt eines Innengewindes ausgebildet. Das Innengewinde 12 erstreckt sich entlang der Längserstreckung eines Schaftes 13, der von der Basis 11 abragt. An der der Basis 11 entfernten Seite des Schaftes 13 ist eine Anbindungsfläche 14 ausgebildet, die im Wesentlichen eben ausgebildet ist. Der Anbindungsfläche 14 gegenüberliegend ist eine Bodenfläche 15 an der Basis 11 ausgebildet, die Anbindungsfläche 11 und die Bodenfläche 15 sind im Wesentlichen parallel zueinander orientiert. Der Schaft 13 ist rotationssymmetrisch, die Zentralbohrung mit dem Innengewinde 12 ist koaxial zur Längserstreckung des Schaftes 13 ausgebildet. Der Schaft 13 ist in dem vorderen Drittel, das zu der Anbindungsfläche 10 gerichtet ist, abgesetzt, das heißt, dass der Schaft 13 dort einen geringeren Durchmesser als im Bereich der Basis 11 aufweist. Die Größe des Absatzes kann variieren, insbesondere ist der Absatz so gewählt, dass auf die Basis 11 aufgelegte Lagen eines Faserverbundwerkstoffes bis zu diesem Absatz reichen oder aber zumindest nicht über den Absatz hinaus in Richtung auf die Anbindungsfläche reichen und gelegt werden. Die Außenkontur des Schaftes 13 kann auch andere Außenkonturen aufweisen, insbesondere eine nicht rotationssymmetrische Außenkontur, um eine Verdrehsicherung zusätzlich zu einer Verdrehsicherung über die unrunde Ausgestaltung der Basis 11 zu ermöglichen.

An dem Befestigungselement 10 können zudem Ausnehmungen, Vorsprünge oder Hinterschnitte angeordnet oder ausgebildet sein, um eine weitere Festlegung auf einer Basislage zur Herstellung eines Grundkörpers für eine orthopädietechnische Einrichtung auszubilden. Die Verwendung des Befestigungselementes 10 im Zusammenhang mit der Herstellung von orthopädietechnischen Einrichtungen wie Orthesen, Prothesen oder anderen orthopädietechnischen Komponenten wird nachfolgend erläutert. Die Basis 11 dient zur Festlegung des Befestigungselementes 10 an einem Grundkörper, während das Formschlusselement 12 dazu dient, dass an dem Befestigungselement 10 weitere Komponenten einer orthopädietechnischen Einrichtung festgelegt werden können, beispielsweise Gelenke, Aktuatoren, Dämpfer oder andere Einrichtungen oder Komponente.

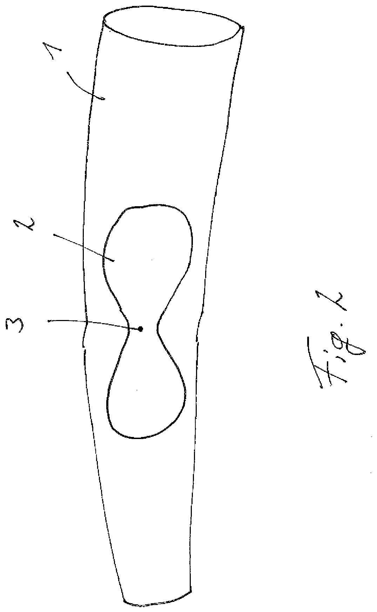

Die Herstellung einer Orthese als eine orthopädietechnische Einrichtung wird anhand der Figuren 2 bis 4 näher erläutert.

Figur 2 zeigt in schematischer Darstellung einen Träger 1, der korrespondierend zu dem Körperteil geformt ist, an dem eine Orthese oder Prothese getragen werden soll. Im dargestellten Ausführungsbeispiel ist der Träger 1 als ein Teil eines Beines mit einem Oberschenkelabschnitt, im Kniegelenk und einem Unterschenkelabschnitt ausgebildet. Alternativ zu einer Ausgestaltung in Beinform kann der Träger auch in Form eines Armes oder eines Armteils ausgebildet sein. Auch ist es möglich und vorgesehen, den Träger 1 in jeglicher anderer Form auszubilden, die notwendig ist, um eine Orthese auszubilden. Sollte eine Prothese hergestellt werden, kann der Träger 1 nur teilweise der Körperform des Patienten oder Prothesennutzers entsprechen, nämlich dort, wo der Stumpf noch vorhanden ist. Der distale Teil des Trägers wird dann modelliert, beispielsweise über ein 3D-Computerverfahren oder auf eine andere Art und Weise.

Auf den Träger 1 wird eine Basislage 2 aufgelegt, die aus einem oder mehreren Zuschnitten gebildet ist. Die Basislage 2 ist bevorzugt aus einem Faserverbundwerkstoff ausgebildet, beispielsweise aus einem Prepreg oder aus einem anderen Faserverbundwerkstoff. Die Basislage 2 im dargestellten Ausführungsbeispiel ist einteilig ausgebildet und erstreckt sich über eine Gelenkachse 3 eines natürlichen oder angenommenen Gelenkes der jeweiligen Gliedmaße. Im dargestellten Ausführungsbeispiel mit dem Träger 1 als Oberschenkelkeil überdeckt die Basislage 2 die Kniegelenkachse 3. Die Basislage 2 ist ausreichend flexibel, um sich an die Oberflächenkontur des Trägers 1 anpassen zu können. Der Träger 1 kann gegenüber der tatsächlichen Kontur der Gliedmaße modifiziert sein, beispielsweise durch Materialzugaben, Glättungen eines 3D-Modells oder ähnliches, um beispielsweise Polsterelemente auf der Innenseite der zu fertigenden Orthese oder Prothese anordnen zu können. Bei einer Prothese kann es notwendig sein, den Prothesenschaft oder die Aufnahmeeinrichtung größer zu wählen, um Liner oder andere Schutzüberzüge aufnehmen zu können, ohne dass eine zu hohe Pressung auf das Körperteil ausgeübt wird.

Die Basislage 2 ist geschlossen ausgebildet, also für Komponenten wie die Befestigungselemente 10, die auf der Basislage 2 aufgelegt werden, undurchlässig. Die Basislage 2 kann auf dem Träger 1 fixiert werden, entweder mechanisch oder über einen Kleber. Die Fixierung erfolgt so, dass die Basislage 2 nach der Fertigstellung der Orthese oder Prothese wieder abnehmbar ist.

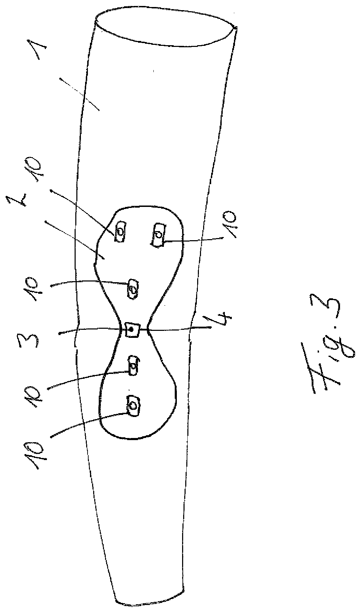

In der Figur 3 ist eine nächste Phase der Herstellung der Orthesenkomponenten gezeigt, bei der auf der lateralen Oberfläche der Basislage 2, also auf derjenigen Oberfläche, die dem Träger 1 abgewandt ist, Befestigungselemente 10 aufgesetzt sind, wie sie bereits in der Figur 1 beschrieben worden sind. Die Befestigungselemente 10 werden mit der Unterseite 15, also derjenigen Fläche der Basis 11, die der Anlagefläche 14 abgewandt ist, aufgesetzt. Dabei werden die Befestigungselemente 10, im dargestellten Ausführungsbeispiel fünf Befestigungselemente 10, von den zwei im distalen Bereich und drei im proximalen Bereich positioniert sind, bevorzugt über eine Positioniereinrichtung auf der Basislage 2 positioniert. Die Positioniereinrichtung wird weiter unten näher erläutert werden. Durch die Positioniereinrichtung sind die Befestigungselemente 10 in definierten Abständen zueinander und von der Gelenkachse 3 entfernt auf der Basislage 2 angeordnet. Die Positioniereinrichtung wird an einer Aufnahme 4 festgelegt oder darauf aufgesetzt, beispielsweise aufgesteckt, aufgeschraubt oder über eine Magnetverriegelung fixiert. Die Aufnahme 4 ist bevorzugt bereits auf dem Träger 1 angeordnet und durchragt eine Ausnehmung in dem Zuschnitt der Basislage 2. Die Aufnahme 4 kann in dem Träger 1 eingearbeitet sein, beispielsweise eingegossen oder eingesetzt. Sie enthält bevorzugt ein Gewinde, eine Hülse oder einen Zapfen, deren Längserstreckung mit der Kniegelenksachse zusammenfällt. Allgemein gesprochen soll die Längserstreckung der Aufnahme 4 mit der Gelenkachse zusammenfallen, um die ein Orthesenoberteil gegenüber einem Orthesenunterteil oder eine Proximalkomponente gegenüber der Distalkomponente der Orthese verschwenkt.

Die Befestigungselemente 10 werden auf der Basislage 2 fixiert, beispielsweise über einen Kleber, eine Spachtelmasse oder aber auch unter Einsatz eines Ausgleichsmaterials. Angestrebt ist, dass sich das Befestigungsmittel wie Spachtel oder Kleber bei der weiteren Behandlung der Orthese nicht verformt. Zur Fertigstellung der Orthese kann diese unter hohen Temperaturen und unter Vakuum ausgehärtet werden, was nicht zu einer Verlagerung der Befestigungselemente 10 oder zu einer Verkippung der Befestigungselemente 10 führen darf.

Nachdem alle Befestigungselemente 10 auf der Basislage 2 befestigt sind, wird die Positioniereinrichtung entfernt, was später erläutert werden wird. Sowohl die Befestigungselemente 10 als auch die Aufnahmeeinrichtung 4 bleiben sicher auf der äußeren oder lateralen Oberfläche der Basislage 2.

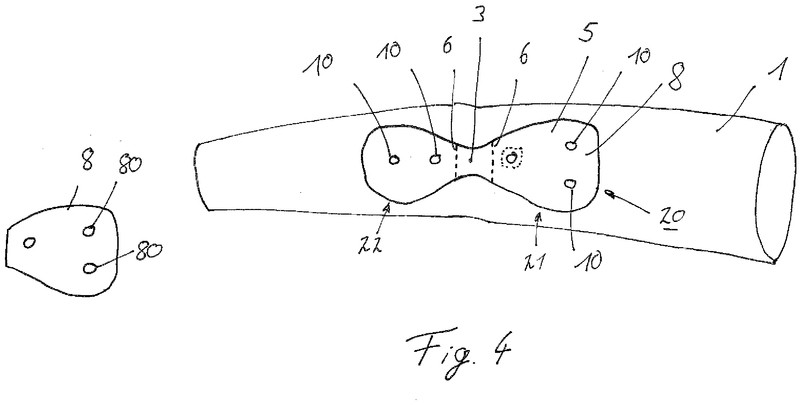

Anschließend wird zumindest eine Lage 8 eines Faserverbundwerkstoffes mit vorgestanzten Ausnehmungen über die Schäfte der Befestigungselemente 10 gelegt, wobei die Ausnehmungen in der Lage 8 aus einem Faserverbundwerkstoff so dimensioniert sind, dass der jeweilige Schaft hindurchragen kann, die Basis 11 jedoch nicht. Dadurch wird die Basis 11 des Befestigungselementes 10 zwischen der Basislage und einer äußeren Faserverbundwerkstofflage 8 eingebettet. In der äußeren Faserverbundwerkstofflage 8 können Solltrennlinien 6 eingearbeitet werden, entlang derer eine Trennung leicht oder erleichtert erfolgen kann. In dem dargestellten Ausführungsbeispiel wird durch zwei Solltrennlinien 6 ein Solltrennbereich ausgebildet, in dem die Gelenkachse 3 und auch die Aufnahmeeinrichtung 4 vor der Ankerplatte liegt. Nach dem Trennen an den Solltrennlinien 6 ergibt sich eine Proximalkomponente 21 und Distalkomponente 22 der Orthese, also eine Oberschenkelschale 21 und eine Unterschenkelschale 22, mit darin einlaminierten Befestigungselementen 10. Das Trennen oder Entfernen des Solltrennbereiches zwischen den Solltrennlinien 6 erfolgt erst, nachdem die Basislage 2 zusammen mit der zumindest einen Faserverbundwerkstofflage 8 auf der Außenseite verklebt und dann aufeinander festgelegt wurde. Dies geschieht beispielsweise nach Anlegen eines Unterdruckes in einem Ofen bei erhöhten Temperaturen. Die Faserverbundwerkstofflagen 8 werden bevorzugt bis zu dem Absatz in dem Schaft 13 aufgelegt. Über den Absatz wird gewährleistet, dass eine ausreichende Materialstärke in dem Bereich der Befestigungselemente vorhanden ist. Eine Faserverbundwerkstofflage 8 als Zuschnitt mit vorgefertigten Ausnehmungen 80, die in dem Durchmesser mit den Schaftdurchmessern der Schäfte 13 und in ihren Positionen den Positionen der Befestigungselemente 10 auf der Basislage 2 entsprechen, ist links in der Figur 4 gezeigt.

Nach dem Aushärten und Abkühlen des Laminatwerkstoffes ist ein Grundkörper 20 vorhanden, mit einer auf der Innenseite durchgehenden Basislage 2, darauf aufgesetzten Befestigungselementen 10 und zumindest einer, vorzugsweise mehreren Lagen Faserverbundwerkstoff 8, die miteinander verbunden sind, so dass die Befestigungselement 10 einlaminiert sind. Der Orthesengrundkörper 20 wird nach dem Aushärten und Abkühlen im Bereich der Solltrennstellen 6 getrennt, beispielsweise zersägt, um die Oberschenkelschale oder proximale Orthesenkomponente 21 von der Unterschenkelschale oder distalen Orthesenkomponente 22 zu trennen. Anschließend werden die Orthesenkomponenten 21, 22 von dem Träger 1 entfernt, gegebenenfalls nachbearbeitet und geschliffen, mit Aufnahmen für Befestigungseinrichtungen wie Gurten versehen und mit den notwendigen und dafür vorgesehenen Anbauteilen wie Gelenkeinrichtungen, Dämpfer oder Polsterungen ausgestattet.

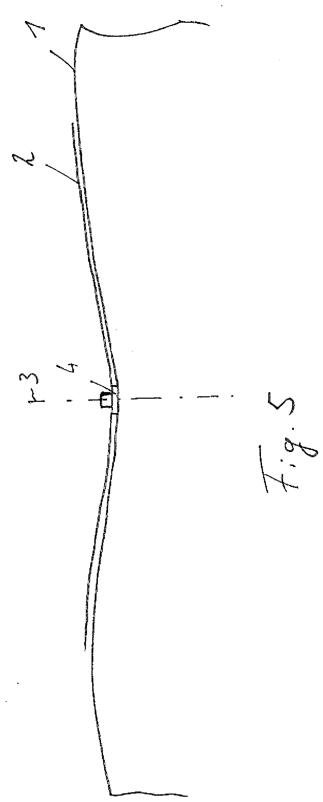

Figuren 5 bis 7 zeigen den Herstellungsablauf einer schematischen Schnittdarstellung. Zunächst wird auf dem Träger 1 die Ankerplatte oder die Aufnahme 4 positioniert, und zwar im Bereich der Gelenkachse des natürlichen Gelenkes oder einer Kompromissachse 3. Anschließend wird die Basislage 2 auf die äußere oder laterale Oberfläche des Trägers 1 gelegt und gegebenenfalls fixiert. Das Material der Basislage 2 kann plastisch verformbar sein und über geringe Rückstellkräfte verfügen, sodass ein möglichst vollständiges Anliegen an der äußeren Oberfläche des Trägers 1 ermöglicht wird. Der Abstand zu dem Träger 1 ist aus Gründen der besseren Sichtbarkeit eingezeichnet.

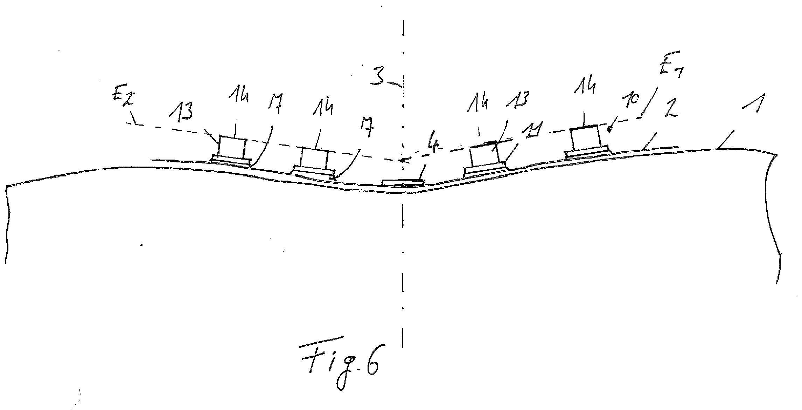

In der Figur 6 ist der Zustand nach dem Aufsetzen der Befestigungselemente 10 auf der lateralen Oberfläche der Basislage 2 gezeigt. Die Befestigungselemente 10 sind über eine Positioniereinrichtung zu der Gelenkachse 3 ausgerichtet auf der Basislage 2 positioniert. Es ist zu erkennen, dass die jeweiligen Basen 11 der Befestigungselemente 10 möglichst nahe an der Oberfläche der Basislage 2 angeordnet werden sollten. Die Verbindung der jeweiligen Unterseite 15 der jeweiligen Basis 11 der Befestigungselemente 10 erfolgt in dem dargestellten Ausführungsbeispiel über eine Spachtelmasse 7, die gleichzeitig Unebenheiten in der Oberfläche der Basislage 2 ausgleicht und dafür sorgt, dass die Befestigungselemente 10 fest auf der Basislage 2 verankert werden.

In Figur 6 ist zu erkennen, dass alle Anbindungsflächen 14 in jeweils einer Ebene E1, E2 liegen, wobei die Ebene E1 für die Befestigungselemente 10 der Proximalkomponente 21 und die Ebene E2 für die Befestigungselemente der Distalkomponente 22 steht. Der Figur 6 ist zu entnehmen, dass die Ebenen E1, E2 in dem dargestellten Ausführungsbeispiel nicht parallel zueinander liegen oder eine gemeinsame Ebene ausbilden. Dies wäre der Fall, wenn sich bei dem Ausführungsbeispiel beispielsweise ein vollständig gerades Bein an der lateralen Seite oder medialen Seite ergeben würde. Dargestellt ist eine mehr natürliche Darstellung, bei der sich eine laterale Auswölbung sowohl des Oberschenkels als auch des Unterschenkels ausgehend von dem Kniegelenk ergibt. Beide Ebenen E1, E2 schneiden sich in dem dargestellten Ausführungsbeispiel in der Gelenkachse 3, sodass sich eine gemeinsame Schnittlinie ergibt, die bevorzugt orthogonal auf der Gelenkachse 3 steht. Es ist auch möglich, dass die Anbindungsflächen nicht exakt in einer Ebene E1, E2 liegen, sondern ein gewisser Höhenversatz vorhanden ist. Ebenfalls ist es möglich, dass sich die Ebenen E1, E2 nicht in der Gelenkachse 3 schneiden, weil sich beispielsweise ein Höhenversatz eingestellt hat. Bevorzugt liegen alle Anbindungsflächen 14 aller Befestigungselemente 10 einer Orthesenkomponente 21, 22 auf einer gemeinsamen Ebene E1, E2. Die Längserstreckungen aller Bohrungen, Zapfen oder Formschlusselemente 12 wie Innengewinde oder Außengewinde in den Befestigungselementen 10 sind parallel bevorzugt zueinander ausgerichtet, bezogen jeweils auf eine Orthosenkomponente. Das heißt, dass alle Längsachsen der Befestigungselemente 10 auf der proximalen Orthesenkomponente 21 bevorzugt parallel zueinander ausgerichtet sind, ebenso wie die Längserstreckungen oder Längsachsen der Befestigungselemente 10 auf einer distalen Orthesenkomponente 22.

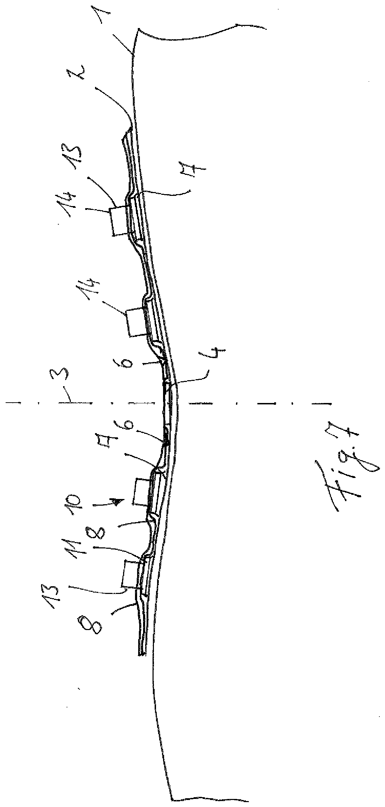

Nach der Festlegung der Befestigungselemente 10 auf der Basislage 2 werden, wie in der Figur 7 gezeigt, mehrere Lagen 8 eines Faserverbundwerkstoffes aufgelegt, beispielsweise harzgetränkte Fasermatten, gegebenenfalls unter Zugabe weiterer Kleber, Härter, Lösungsmittel oder dergleichen. Die Lagen 8 des Faserverbundwerkstoffes oder der Faserverbundwerkstoffe können in unterschiedlichen Orientierungen aufgelegt werden, um die Basen 11 der Befestigungselemente 10 einzulaminieren. Dazu sind in den Zuschnitten der Faserverbundwerkstofflagen 8 Ausnehmungen 80 oder Ausstanzungen ausgebildet, die der Form und dem Durchmesser der jeweiligen Schäfte 13 entsprechen. Da die Basen 11 größer als die Durchmesser der Schäfte 13 sind, kann nach dem Verbinden der Faserverbundwerkstofflagen 8 mit der Basislage 2 kein Befestigungselement 10 mehr aus der jeweiligen Orthesenkomponente 21, 22 entfernt werden. Aufgrund der unrunden Ausgestaltung der Basis 11 ist eine verdrehgesicherte Festlegung aller Befestigungselemente 10 gesichert. Um die Verdrehsicherung zu erhöhen, können Vorsprünge, Haken, Hinterschneidungen oder Ähnliches vorgesehen sein, damit die Befestigungselemente 10 sich nach der Fertigstellung der Orthesenkomponenten 21, 22 nicht verdrehen können.

Alle Anbindungsflächen 14 sind nicht von einer Faserverbundwerkstofflage 8 abgedeckt, um die Zugänglichkeit zu den Formschlusselementen 12 zu gewährleisten und eine definierte Anlage der zu montierenden Komponenten zu gewährleisten. Um Verunreinigungen des Formschlusselementes 12 zu vermeiden, kann diese gesichert werden. Bei der Ausführungsform gemäß der Figur 1, bei der das Formschlusselement 12 als ein Innengewinde ausgebildet ist, kann dieses beispielsweise über eine Schraube erreicht werden, die nach der Fertigstellung der Orthesenkomponente herausgeschraubt wird. Ist das Formschlusselement 12 als ein Außengewinde ausgebildet, kann eine Schraubkappe aufgeschraubt werden, um das Gewinde zu schützen. Korrespondierendes gilt für andere Formschlusselemente, wie Zapfen, Bohrungen oder dergleichen. Nach der Auflage der Faserverbundwerkstofflagen 8 wird unter Anlegung eines Vakuums und Aufbringung erhöhter Temperaturen auf dem Träger 1 der Orthesengrundkörper 20 fertiggestellt. Die Solltrennstellen 6 sind proximal und distal an der Gelenkachse 3 ausgebildet, beispielsweise durch Eindrückungen oder durch Schnitte in den Faserverbundwerkstofflagen 8 oder einfach durch keine oder weniger Faserverbundwerkstofflagen 8 in dem Bereich zwischen den Solltrennstellen 6.

Nach dem Aushärten und Trennen der Orthesenkomponenten 21, 22 voneinander können andere Komponenten an den Befestigungselementen 10 festgelegt werden.

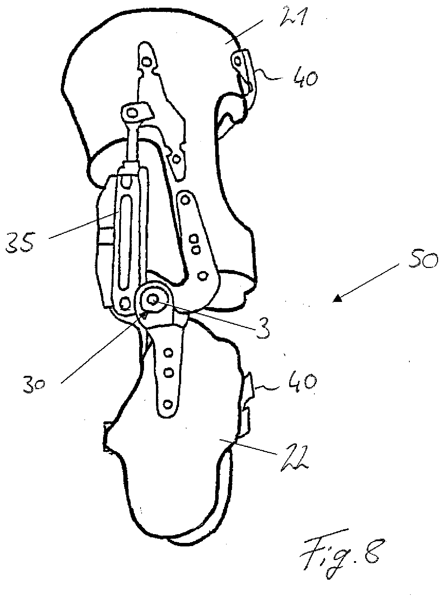

In der Figur 8 ist eine Variante einer Kniegelenksorthese gezeigt, bei der Proximalkomponente 21 als Oberschenkelschale und die Distalkomponente 22 als Unterschenkelschale ausgebildet ist. An beiden Orthesenkomponenten 21, 22, sind Befestigungseinrichtungen 40 angeordnet, die als Gurte ausgebildet sind, um Orthese 50 an einem Bein festzulegen. Eine Gelenkeinrichtung 30 mit einem hydraulischen Aktor 35 ist an den nicht mehr sichtbaren Befestigungselementen festgelegt, beispielsweise über Schrauben. Die Gelenkeinrichtung 30 hat ihre Schwenkachse im Bereich der Gelenkachse 3 des natürlichen Gelenkes. Die Position der Gelenkachse 3 auf der Gelenkeinrichtung 30 ist durch die exakte Positionierung der Befestigungselemente relativ zu der Gelenkachse 3 des natürlichen Gelenkes über eine Positioniereinrichtung gesichert. Die Form der Orthesenkomponenten 21, 22 in Gestalt der Orthesenschalen ist sehr gut und individuell an die Form des jeweiligen Orthesennutzers angepasst. Die Herstellung der Orthese kann ohne vorherige Anordnung der Gelenkeinrichtung 30 oder einer hydraulischen Komponente 35 an den Orthesenkomponenten 21, 22 erfolgen, was im Hinblick auf die bei der Fertigung auftretenden hohen Temperaturen und Unterdrücke insbesondere für elektronische Steuerungen außerordentlich vorteilhaft ist. Durch die Art und Weise der Fertigung wird eine Restriktion der zu verwendenden Anbauteile wie Dämpfer, Steuerung oder dergleichen verhindert.

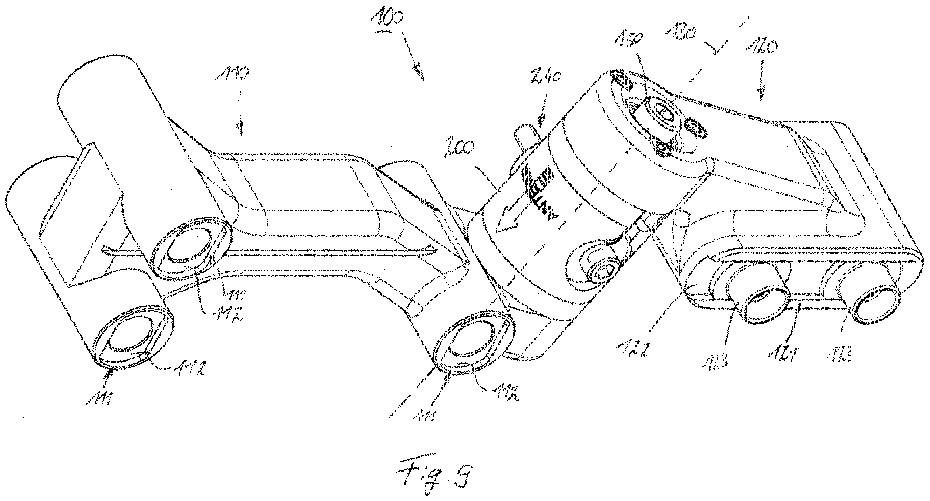

Figur 9 zeigt in einer perspektivischen Ansicht eine Positioniereinrichtung 100 zur Positionierung und Ausrichtung von nicht dargestellten Befestigungselementen 10, wie sie in der Figur 1 beispielhaft erläutert sind. Die Positioniereinrichtung 100 weist einen Zentralkörper 200 auf, an dem zwei Halter 110, 120 schwenkbar um eine Schwenkachse 130 angeordnet sind. Ein erster Halter 110 ist in dem dargestellten Ausführungsbeispiel für die Zuordnung und Anordnung der Befestigungselemente 10 auf der proximalen Orthesenkomponente 21 vorgesehen, während der zweite Halter 120 für die Befestigungselemente 10 auf der distalen Orthesenkomponente 22 vorgesehen ist. Beide Halter 110, 120 weisen Aufnahmeeinrichtungen 111, 121 auf, die als Hülsen ausgebildet sind, die Durchgangsbohrungen aufweisen, durch die Fixierelemente 123 hindurchführbar sind. In der Figur 9 sind die Fixierelemente 123 nur an dem zweiten Halter 120 gezeigt. An den Aufnahmeeinrichtungen 111, 121 sind Anlageflächen 112, 122 für die Oberseite der Basis 11 der Befestigungselemente 10 ausgebildet. Die Oberseite der Basis 11 ist diejenige Seite der Basis 11, die Unterseite 15 gegenüberliegt. In dem dargestellten Ausführungsbeispiel sind alle Anlagenflächen 112, 120 an einem gemeinsamen Halter 110, 120 in einer gemeinsamen Ebene angeordnet, um sicherzustellen, dass alle Befestigungselemente 10 in einer gemeinsamen Ebene liegen, wenn sie an dem jeweiligen Halter 110, 120 angeordnet und durch die Fixierelemente 123 dort festgelegt sind.

An dem Zentralkörper 200 ist eine Fixiereinrichtung 240 in Gestalt einer Schraube angeordnet, über die der Zentralkörper 200 an der Aufnahme 4, die an dem Träger 1 oder der Basislage 2 fixiert ist, festgelegt wird. Die Längserstreckung der Fixiereinrichtung 240 verläuft senkrecht zu der Schwenkachse 130 und schneidet diese vorzugsweise, sodass die Längsachse der Fixiereinrichtung 240 orthogonal auf der Schwenkachse 130 steht. Die Längserstreckung der Fixiereinrichtung 240 fluchtet bevorzugt mit der Längsachse 3 der Gelenkeinrichtung und der natürlichen Gelenkachse oder der Kompromissachse für das natürliche Gelenk. Befinden sich alle Anlageflächen 112, 122 in parallelen Ebenen oder in einer gemeinsamen Ebene, je nachdem, wie die Ebenen der Anlageflächen 112, 120 angeordnet sind, befindet sich die Positioniereinrichtung 100 in einer Ausgangsstellung. Aus dieser Ausgangsstellung können sowohl der erste Halter 110 als auch der zweite Halter 120 um einen eingeschränkten Winkelbereich, beispielsweise um +/- 10°, um die Schwenkachse 130 verschwenkt werden. Gelenkeinrichtungen 30 oder auch andere Anbauteile können hinsichtlich eines möglichen Winkelversatzes ihrer Anbindungsstellen empfindlich sein. Durch die Positioniereinrichtung 100 ist es möglich, neben einer exakten Positionierung der Befestigungselemente 10 relativ zueinander und zu einer Gelenkachse 3 um eine Gelenkeinrichtung 30 diesen maximalen Winkelversatz Rechnung zu tragen. Es besteht die Möglichkeit, den Winkelversatz der beiden Halter 110, 120 vorab größenmäßig festzulegen. Ist beispielsweise ein maximaler Versatz der Ebenen der Anbindungsflächen 14 von 10° zulässig, kann dieser maximale Winkelbereich mit der Positioniereinrichtung 100 eingestellt werden. Wird dann ausgehend von der Ausgangsstellung zunächst der erste Halter 110 auf die Basislage 2 aufgelegt und benötigt eine Verschwenkung in Lateralrichtung um 3°, ausgehend von der Ausgangsstellung, steht für den zweiten Halter 120 ein maximaler Verschwenkbereich um weitere 7° in Lateralrichtung zur Verfügung. Ist bei einer solchen Maximaleinstellung eine zufriedenstellende Orientierung der Unterseiten 15 der Basen 11 aller Befestigungselemente 10 nicht möglich, muss die gesamte Positioniereinrichtung 100 weiter lateral versetzt werden oder aber über eine Ausgleichsmasse oder über eine Spachtelmasse eine Festlegung der Befestigungselemente 10 auf der Basislage erfolgen.

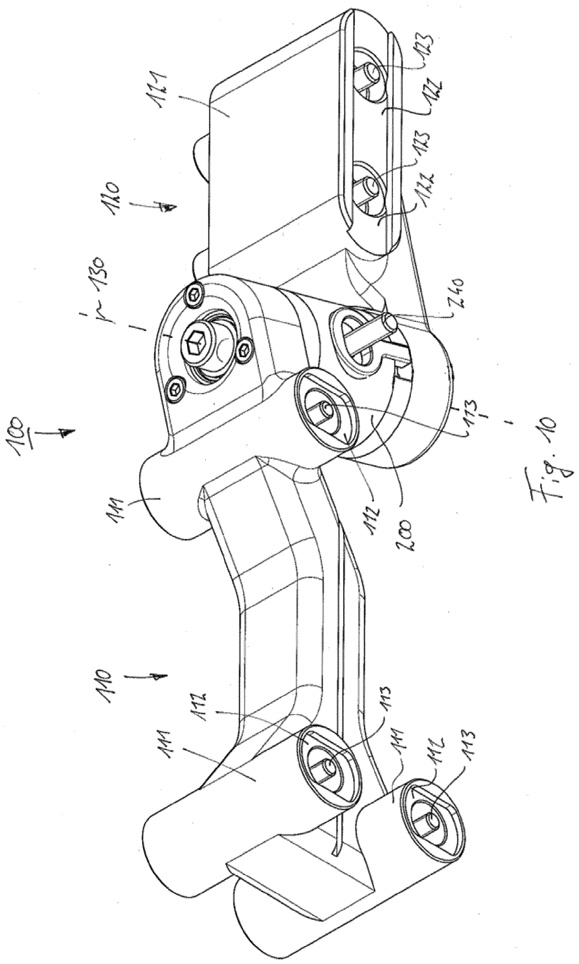

Die Positioniereinrichtung 100 ist klappsymmetrisch ausgebildet. In der Figur 9 ist beispielsweise eine Oberseite zu erkennen, während in der in Figur 10 die Unterseite dargestellt ist. Der Vergleich der Figuren 9 und 10 zeigt, dass auf beiden Seiten der Aufnahmeeinrichtungen 111, 121 gleiche Aufnahmen für die Befestigungselemente 10 ausgebildet sind. Die Fixiereinrichtung 240 kann aus dem Zentralkörper 200 herausgenommen und umgekehrt wieder eingeführt werden, sodass die Positioniereinrichtung 100 sowohl für ein rechtes Bein als auch für ein linkes Bein sowie für eine mediale als auch laterale Positionierung auf Basislagen 2 geeignet ist.

In der Figur 10 sind die Fixierelemente 113 in Gestalt von Schrauben in allen Aufnahmeeinrichtungen 111 gezeigt. Die Innengewinde 12 gemäß der Figur 1 sind korrespondierende zu Außengewinden an den Fixierelementen 113 ausgebildet, sodass die Montage dergestalt erfolgt, dass in jeder Aufnahmeeinrichtung 111 mit den Anbindungsflächen voran der Schaft 13 in die Bohrungen der hülsenartigen Aufnahmeeinrichtungen 111 eingeführt werden. Über die Fixierelemente 113 werden die Befestigungselemente fixiert. Es ist zu erkennen, dass die Form der Anlageflächen 112 der Aufnahmeeinrichtung 111 der Form und Kontur der Basen 11 entsprechen, sodass eine definierte Zuordnung und Orientierung jedes Befestigungselementes 10 an dem jeweiligen Halter 110, 120 erfolgt. In dem zweiten Halter 120 ist eine nutenartige Führung für die beiden Basen 11 der beiden Befestigungselemente 10 vorgesehen. Hierin können weitere Einlageelemente wie Schienen oder Verstärkungselement oder Abstandshalter aufgenommen werden, die ebenfalls einlaminiert werden können. Nach der Fixierung der Befestigungselemente 10 innerhalb der Aufnahmeeinrichtungen 111, 121 wird die Positioniereinrichtung 100 mit der Fixiereinrichtung 240 in der Aufnahme 4 festgelegt. Eine Zentralschraube 150 entlang der Schwenkachse 130 hält die beiden Halter 110, 120 einer definierten Stellung zueinander, bevorzugt in der Ausgangsstellung, bei der alle Unterseiten 15 der Befestigungselemente 10 in einer gemeinsamen Ebene oder zumindest in parallelen Ebenen zueinander ausgerichtet sind. Wird die Fixierung durch die Zentralschraube 150 gelöst, können die beiden Halter 110, 120 um die Schwenkachse 130 in dem vorgegebenen Winkelbereich verschwenken.

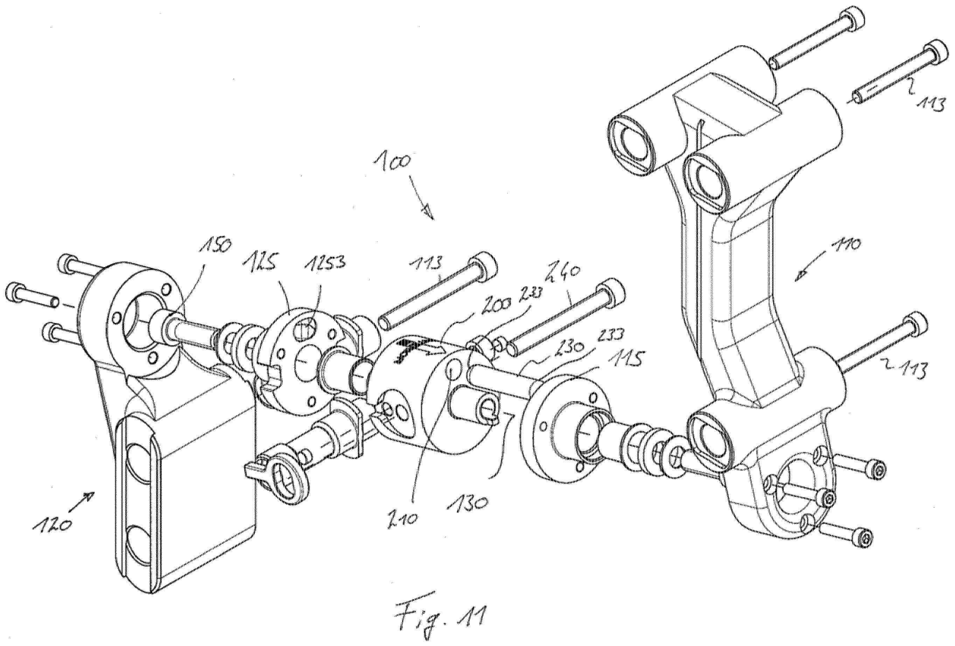

Figur 11 eine Explosionsdarstellung der Positioniereinrichtung 100 mit dem Zentralkörper 200, der Fixiereinrichtung 240, die durch eine Bohrung innerhalb des Zentralkörpers 200 hindurchgeht und die Schwenkachse 130 orthogonal schneidet. Die Fixierelemente 113 sind zu erkennen, ebenso die beiden Halter 110, 120 und die Zentralschraube 150, die sich entlang der Schwenkachse 130 erstreckt. Innerhalb des Zentralkörpers 200 ist ebenfalls ein Anschlagelement 230 in einer Bohrung 210 in dem Zentralkörper 200 längsverschieblich gelagert. Die Bohrung 210 erstreckt sich parallel zu der Schwenkachse 130.

An den Haltern 110, 120 sind über drei Schrauben jeweils ein Gegenstück 115, 120 mit Anlagenflächen 1153, 1253 angeordnet, die mit den Anlagenflächen 233 an den beiden Enden des Anschlagelementes 230 wechselwirken. Die Wechselwirkung wird nachfolgend erläutert. Die Gegenstücke 115, 125 sind in dem dargestellten Ausführungsbeispiel ortsfest an dem jeweiligen Halter 110, 120 gelagert. Es besteht auch die Möglichkeit, beispielsweise durch Langlöcher eine Verdrehbarkeit der Gegenstücke 115, 125 an dem jeweiligen Halter 110, 120 zu ermöglichen. Über die Verdrehung der Gegenstücke 115, 125 kann eine Stellung des Winkelbereiches erfolgen, eine Vergrößerung des maximalen Winkelbereiches kann beispielsweise über einen Austausch der Gegenstücke 115, 125 erfolgen. Ebenfalls ist es möglich, beispielsweise durch Verstellschrauben die Position der Anschlagflächen 1153, 1253 zu verändern, um den Winkelbereich, um den der erste Halter 110 relativ zu dem zweiten Halter 120 um die Schwenkachse 130 verschwenkt werden kann, einzustellen. Dazu können Verstellschrauben in die Ausnehmungen in dem Gegenstück 115, 125 eingeschraubt oder herausgeschraubt werden.

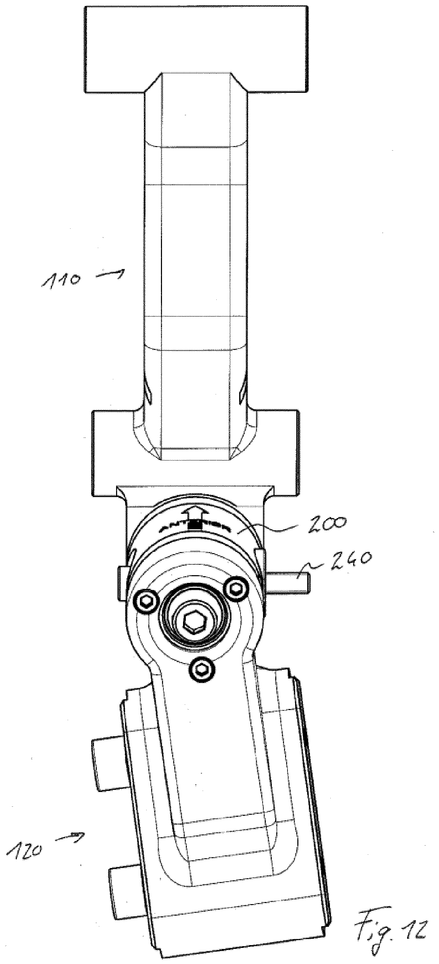

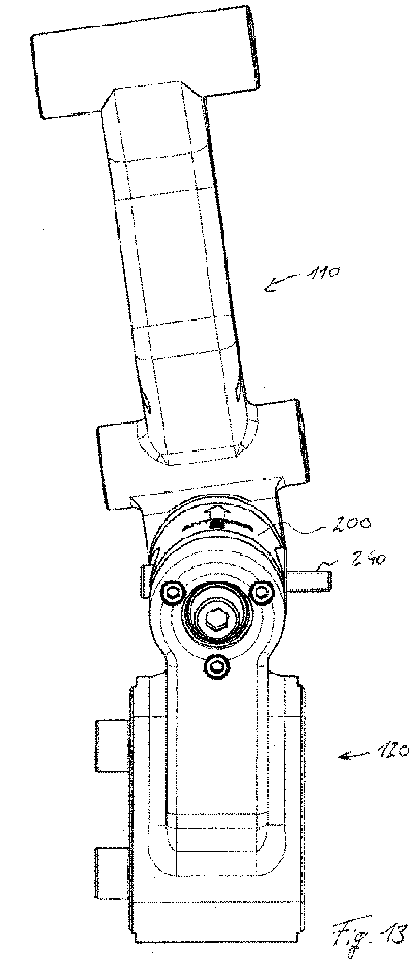

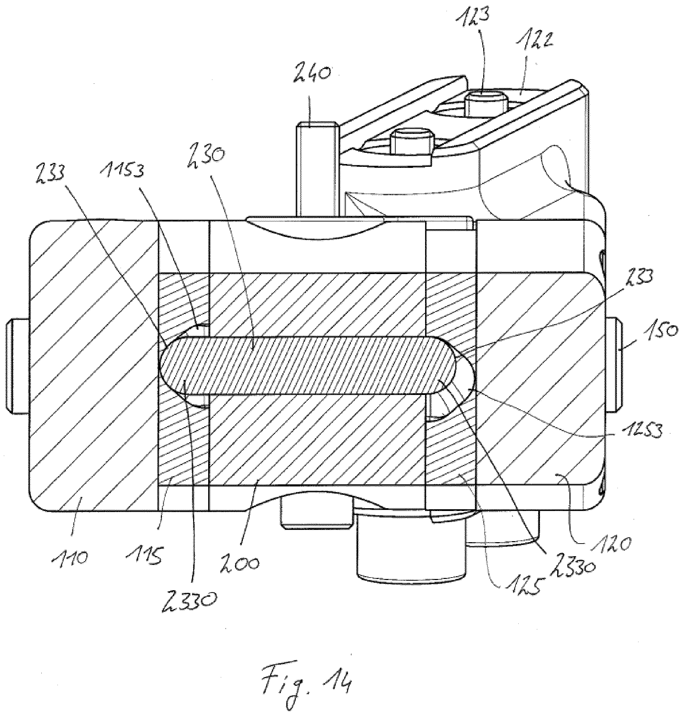

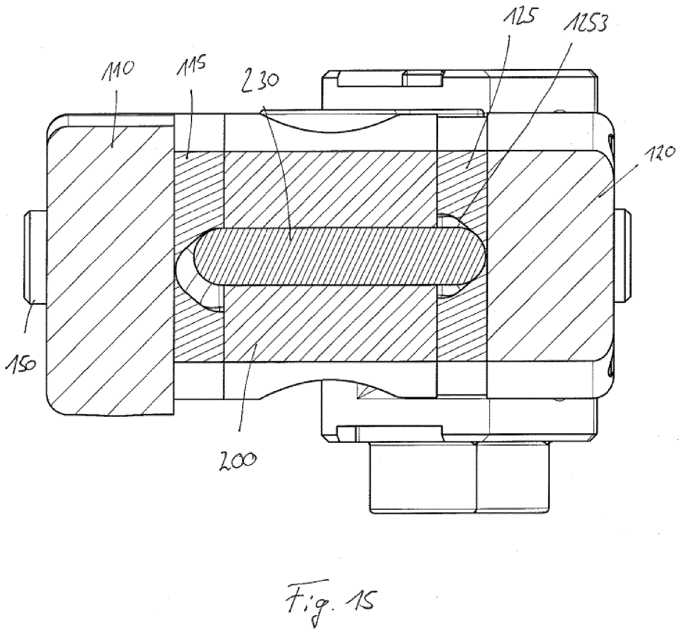

Figuren 12 und 13 zeigen die Positioniereinrichtung 100 in der jeweils gleichen Ansicht. In der Figur 12 ist ausgehend von der Ausgangsstellung der zweite Halter 120 maximal entgegen dem Uhrzeigersinn um die Schwenkachse 130 verschwenkt. In der Figur 13 ist der erste Halter 110 maximal entgegen dem Uhrzeigersinn, ausgehend von der Ausgangsstellung, verschwenkt. In beiden Stellungen der Figuren 12 und 13 ist jeweils der maximale Verschwenkbereich erreicht. Korrespondierende Schnittdarstellungen zu den Figuren 12 und 13 sind die Figuren 14 und 15.

Die Figur 14 zeigt einen Schnitt durch das Zentralstück 200 in dem Bereich des Anschlagelementes 230. Das Anschlagelement 230 sieht in der Schnittdarstellung wie eine Passfeder aus, die verschieblich innerhalb des Zentralstückes 200 angeordnet ist. In der Figur 14 befindet sich ein gerundeter Endbereich 2330 mit entsprechenden Anlageflächen 233 in Anschlag mit einer korrespondierend ausgebildeten Anlagefläche 1153 in einer Ausnehmung in dem Gegenstück 115. Das Gegenstück 115 ist drehstarr an dem ersten Halter 110 verbunden. Das Gegenstück 115 befindet sich in der Ausgangsstellung, in der der erste Halter 110 entsprechend orientiert ist. In dieser Ausgangsstellung kann das Anschlagselement 230 maximal nach links parallel zur Schwenkachse 130 verschoben werden. Dadurch wird das rechte Ende des Anschlagelements 230 aus dem Freiraum innerhalb des Gegenstückes 125 des zweiten Halters 120 gebracht, sodass der zweite Halter 120 sich maximal in beide Richtungen bewegen kann. In dem dargestellten Ausführungsbeispiel wurde der Halter 120 um die Schwenkachse nach oben verschwenkt, sodass die Anlagefläche 1253 an der gerundeten Anlagefläche 233 des rechten Endes des Anschlagelements 230 anliegt. Würden sich beide Halter 110, 120 in der Ausgangsstellung befinden und das Anlagestück 230 in der Mitte befinden, würden beide Halter 110, 120 um den gleichen Winkel um die Schwenkachse 130 verschwenken können, bis sie zu einer Anlage der Anlageflächen 233, 1153, 1253 kommen würden. Je weiter das Anschlagelement 230 in die eine oder andere Richtung verschoben wird, desto mehr vergrößert oder verringert sich der mögliche Verschwenkbereich des anderen Halters in der einen oder anderen Verschwenkrichtung. Sind die Anlageflächen 1153, 1253 der Gegenstücke 115, 125 nicht gleichgeformt oder symmetrisch, können sich unterschiedliche Winkeleinstellmöglichkeiten ergeben. Neben einer gerundeten Ausgestaltung der Anlageflächen 233, 1153, 1253 können diese auch andere Formen aufweisen.

In der Figur 15 ist die umgekehrte Position gemäß Figur 13 gezeigt, das Anschlagelement 230 ist maximal nach rechts verschoben, wodurch das rechte Ende des Anschlagelementes 230 in die Ausnehmung in dem Gegenstück 125 und damit an die Anlagenflächen 1253 anstößt. Dadurch ergibt sich eine maximale Verschwenkbarkeit des ersten Halters 115 um die Schwenkachse 130.

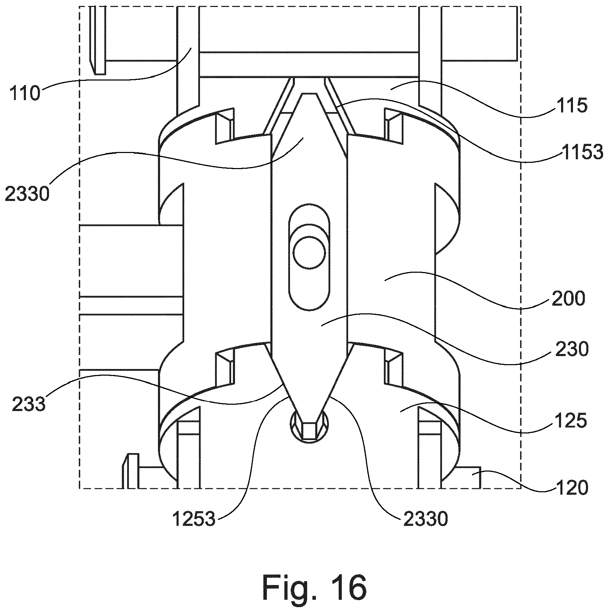

Eine alternative Ausführungsform des Anschlagelementes 230 ist in der Figur 16 gezeigt, bei der statt einer gerundeten Ausgestaltung der beiden Endstücke 2330 eine gerade, konische Ausgestaltung der Endstücke 2330 und der Anlageflächen 233 vorhanden ist. Eine korrespondierende konische Ausgestaltung der Anlageflächen 1153, 1253 ermöglicht eine große Anlagefläche und damit eine geringe Flächenpressung. Die Längsverschieblichkeit des Anschlagelementes 230 ermöglicht eine einfache Verstellung. In dem Anschlagelement 230 kann ein Langloch ausgebildet sein, durch das eine Schraube oder eine Verschiebungsbegrenzung eingeführt werden kann, um den Verstellbereich der Halter 110, 120 zueinander zu begrenzen. Das Anschlagelement 230 kann in der jeweils gewünschten Stellung fixiert werden. Die schräge Ausgestaltung der Anlageflächen 233, 1153, 1253 bedingt eine Verschiebung des Anschlagelementes 230 bei Kontakt entlang der Verschieberichtung in Richtung auf den gegenüberliegenden Halter, wodurch sich dessen Verstellwinkel in beiden Verschwenkrichtungen verändert. Der jeweilige Verschwenkbereich der Halter kann in Abhängigkeit der Stellungen der Halter zueinander innerhalb eines vorgegebenen Winkelbereiches verändert werden. Es ist vorgesehen, dass die Halter 110, 120 in der jeweils gefundenen, optimalen Stellung, in der die Befestigungselemente 10 auf der Basislage 2 aufgesetzt sind, festgehalten werden. Dies kann beispielsweise durch eine Klemmung durch die Zentralschraube 115 erfolgen.

## Patentansprüche

1. Verfahren zum Herstellen von Orthesen- oder Prothesenkomponenten zur Aufnahme von oder Befestigung an einem Körperteil mit den Schritten:
a. Aufbringen einer Basislage (2) auf einem zu der Körperteilform korrespondierend geformten Träger (1),
b. Anordnen mehrerer Befestigungselemente (10) mit einer Basis (11) und einem von der Basis (11) abstehenden Formschlusselement (12) auf der Basislage (2) in definierten Positionen zueinander, wobei die Basis (11) der Befestigungselemente (10) auf der Basislage (2) aufliegt oder der Basislage (2) zugewandt ist,
c. Auflegen zumindest einer Lage (8) eines Faserverbundwerkstoffes auf die Basislage (2) und Einbetten der Basis (11), wobei das Formschlusselement (12) von der der Basislage (2) abgewandten Seite aus zugänglich bleibt, und
d. Aushärten der zumindest einen Faserverbundwerkstofflage (8), **dadurch gekennzeichnet, dass** nach dem Aushärten die Befestigungselemente (10) verdrehgesichert oder unlösbar in der zumindest einen Faserverbundwerkstofflage (8) eingebettet sind.

2. Verfahren nach Anspruch 1, **dadurch gekennzeichnet,**
**dass** die Basislage (2) mit der zumindest einen Faserverbundwerkstofflage (8) verbunden und die Basis (11) der Befestigungselemente (10) zwischen der Basislage (2) und der zumindest einen Faserverbundwerkstofflage (8) einlaminiert wird.

3. Verfahren nach Anspruch 1 oder 2, **dadurch gekennzeichnet, dass** das Formschlusselement (12) an oder in einem Schaft (13) ausgebildet oder angeordnet ist und der Schaft (13) teilweise in die zumindest eine Faserverbundwerkstofflage (8) eingebettet wird und teilweise aus der Faserverbundwerkstofflage (8) herausragt.

4. Verfahren nach Anspruch 3, **dadurch gekennzeichnet, dass** die Schäfte (13) mehrerer Befestigungselemente (10), insbesondere aller Befestigungselemente (10), parallel zueinander ausgerichtet werden.

5. Verfahren nach einem der voranstehenden Ansprüche, **dadurch gekennzeichnet, dass** an den Befestigungselementen (10) zumindest eine Anbindungsfläche (14) angeordnet oder ausgebildet ist, die von der Basis (11) beabstandet ist und nicht von der zumindest einen Faserverbundwerkstofflage (8) abgedeckt wird.

6. Verfahren nach einem der voranstehenden Ansprüche, **dadurch gekennzeichnet, dass** die Basis (11) auf der Basislage (2) fixiert wird.

7. Verfahren nach einem der voranstehenden Ansprüche, **dadurch gekennzeichnet, dass** der Träger (1) als ein Positivmodel des Körperteils ausgebildet wird und das Aushärten der zumindest einen Faserverbundwerkstofflage (8) an dem Träger (1) erfolgt.

8. Verfahren nach einem der voranstehenden Ansprüche, **dadurch gekennzeichnet, dass** in der zumindest einen aufgelegten Faserverbundwerkstofflage (8) Ausnehmungen ausgebildet sind, durch die Teile der Befestigungselemente (10) hindurchgeführt werden.

9. Verfahren nach einem der voranstehenden Ansprüche, **dadurch gekennzeichnet, dass** die Befestigungselemente (10) verdrehgesichert und unlösbar an den Orthesen- oder Prothesenkomponenten festgelegt werden.

10. Verfahren nach einem der voranstehenden Ansprüche, **dadurch gekennzeichnet, dass** die Befestigungselemente (10) auf der dem Körperteil zugewandten Seite der Orthesen- oder Prothesenkomponente mit der Basislage (2) vollständig abgedeckt werden.

11. Verfahren nach einem der voranstehenden Ansprüche, **dadurch gekennzeichnet, dass** Anbindungsflächen (14), die an den Befestigungselementen (10) beabstandet zu der Basis (11) positioniert sind, aller Befestigungselemente (10) in einer Ebene oder in einem Bereich zwischen zwei parallelen Ebene vorpositioniert werden und ein vorhandener Abstand zwischen die Basen (11) zu der Basislage (2) über ein Ausgleichselement oder eine Ausgleichsmasse (7) aufgefüllt wird.

12. Verfahren nach einem der voranstehenden Ansprüche, **dadurch gekennzeichnet, dass** die Befestigungselemente (10) in vorbestimmten Positionen zueinander und zu einer Gelenkachse (3) eines Gelenkes des Körperteils vorpositioniert und auf der Basislage (2) angeordnet oder dieser zugeordnet werden.

13. Verfahren nach Anspruch 12, **dadurch gekennzeichnet, dass** die Befestigungselemente (10) an einem Halter (110, 120) oder einer Positioniereinrichtung (100) angeordnet und zueinander vorpositioniert werden, bevor sie auf der Basislage (2) angeordnet oder dieser zugeordnet werden.

14. Verfahren nach einem der voranstehenden Ansprüche, **dadurch gekennzeichnet, dass** zur Ausbildung einer Orthese mit einer Distalkomponente (22) und einer Proximalkomponente (21) die zumindest eine Faserverbundwerkstofflage (8) in einem Bereich, der dem entspricht, in dem ein natürliches Gelenk befindlich ist, auf dem Träger (1) angeordnet und zur Ausbildung eines Grundkörpers (20) ausgehärtet wird, wobei die Befestigungselemente (10) vor dem Aushärten proximal und distal einer Gelenkachse (3) angeordnet und in der zumindest einen Faserverbundwerkstofflage (8) eingebettet werden und dass nach dem Aushärten die zumindest eine Faserverbundwerkstofflage (8) oder ein nicht von der Faserverbundwerkstofflage (8) abgedeckter Bereich der Basislage (2) des Grundkörpers (20) in dem Bereich der Gelenkachse (3) durchtrennt wird, um aus dem Grundkörper (20) die Distalkomponente (22) und die Proximalkomponente (21) der Orthese auszubilden.

15. Verfahren zum Herstellen einer Orthese, bei dem die Orthesenkomponenten (21, 22) gemäß dem Verfahren nach Anspruch 14 hergestellt worden sind und Festlegen einer Gelenkeinrichtung (30) an den Befestigungselementen (10).

16. Orthesen- oder Prothesenkomponente zur Aufnahme von oder zur Befestigung an einem Körperteil, die Orthesen- oder Prothesenkomponente (21, 22) weist eine Basislage (2) auf, die auf einem zu der Körperteilform korrespondierend geformten Träger (1) anordenbar ist, auf der Basislage (2) ist zumindest ein Befestigungselement (10) mit einer Basis (11) und einem von der Basis (11) abstehenden Formschlusselement (12) angeordnet, wobei die Basis (11) auf der Basislage (2) aufliegt oder ihr zugewandt ist und das Befestigungselement (10) in zumindest einer Lage Faserverbundwerkstoff (8) eingebettet ist und das Formschlusselement (12) von der der Basislage (2) abgewandten Seite aus nach dem Aushärten der zumindest einen Faserverbundwerkstofflage (8) zugänglich bleibt, wobei
mehrere Befestigungselemente (10) eingebettet sind, die einen Schaft (13) mit oder als Formschlusselement (12) und zumindest eine nicht eingebettete Anbindungsfläche (14) aufweisen, wobei die Schäfte (13) parallel zueinander ausgerichtet sind, **dadurch gekennzeichnet, dass** die Befestigungselemente (10) verdrehgesichert oder unlösbar in der zumindest einen Faserverbundwerkstofflage (8) eingebettet sind.

17. Orthesen- oder Prothesenkomponente nach Anspruch 16, **dadurch gekennzeichnet, dass** Anbindungsflächen (14) parallel zueinander ausgerichtet sind.

18. Orthesen- oder Prothesenkomponente nach Anspruch 16 oder 17, **dadurch gekennzeichnet, dass** die Anbindungsflächen (14) in einer Ebene (E1, E2) oder zwischen zwei parallel zueinander ausgerichteten Ebenen versetzt oder in einem Winkel zueinander angeordnet sind.

19. Orthesen- oder Prothesenkomponente nach einem der Ansprüche 16 bis 18, **dadurch gekennzeichnet, dass** die Basislage (2) aus einem Faserverbundwerkstoff ausgebildet ist und eine geschlossene Oberfläche auf der dem Körperteil zugewandten Seite ausbildet.

20. Orthesen- oder Prothesenkomponente nach einem der Ansprüche 16 bis 19, **dadurch gekennzeichnet, dass** die Befestigungselemente (10) eine unrunde Basis (11) und/oder Vorsprünge und/oder Ausnehmungen (16) zur Verdrehsicherung aufweist.

21. Orthesen- oder Prothesenkomponente nach einem der Ansprüche 16 bis 20, **dadurch gekennzeichnet, dass** an oder in dem Formschlusselement (12) ein Gewinde angeordnet oder ausgebildet ist.

22. Orthesen- oder Prothesenkomponente nach einem der Ansprüche 16 bis 21, **dadurch gekennzeichnet, dass** daran Befestigungseinrichtungen (40) zur Festlegung an einem Körperteil angeordnet sind.

23. Orthese aus mehreren Orthesenkomponenten nach einem der Ansprüche 16 bis 22 und zumindest einer an den Befestigungselementen (10) festgelegten Gelenkeinrichtung (30).

24. Orthese aus mehreren Orthesenkomponenten nach einem der Ansprüche 16 bis 22, die miteinander ein natürliches Gelenk übergreifend verbunden sind und zumindest eine Solltrennstelle (6) oder einen Solltrennbereich aufweisen, in der oder in dem die Gelenkachse (3) des natürlichen Gelenkes liegt.

## Claims

1. A method for the production of orthosis or prosthesis components for receiving or for fastening to a body part, said method having the steps of:
a. applying a base layer (2) to a support (1) which is shaped corresponding to the shape of the body part,
b. arranging a plurality of fastening elements (10), having a base (11) and a form-fit element (12) protruding from the base (11), on the base layer (2) in defined positions relative to each other, wherein the base (11) of the fastening elements (10) bears on the base layer (2) or faces toward the base layer (2),
c. placing at least one layer (8) of a fiber composite material on the base layer (2) and embedding the base (11), wherein the form-fit element (12) remains accessible from the side facing away from the base layer (2), and
d. curing the at least one fiber composite material layer (8), **characterized in that** after the curing the fastening elements (10) are embedded non-releasably or in a manner secure against rotation in the at least one layer (8) of fiber composite material.

2. The method as claimed in claim 1, **characterized in that** the base layer (2) is connected to the at least one fiber composite material layer (8), and the base (11) of the fastening elements (10) is laminated in between the base layer (2) and the at least one fiber composite material layer (8).

3. The method as claimed in claim 1 or 2, **characterized in that** the form-fit element (12) is formed or arranged on or in a shaft (13), and the shaft (13) is partially embedded in the at least one fiber composite material layer (8) and partially protrudes from the fiber composite material layer (8).

4. The method as claimed in claim 3, **characterized in that** the shafts (13) of a plurality of fastening elements (10), in particular of all the fastening elements (10), are oriented parallel to each other.

5. The method as claimed in one of the preceding claims, **characterized in that** at least one binding surface (14) is arranged or formed on the fastening elements (10), which binding surface (14) is spaced apart from the base (11) and is not covered by the at least one fiber composite material layer (8).

6. The method as claimed in one of the preceding claims, **characterized in that** the base (11) is fixed to the base layer (2).

7. The method as claimed in one of the preceding claims, **characterized in that** the support (1) is configured as a positive model of the body part, and the curing of the at least one fiber composite material layer (8) takes place on the support (1).

8. The method as claimed in one of the preceding claims, **characterized in that** recesses are formed in the at least one applied fiber composite material layer (8), through which recesses parts of the fastening elements (10) are guided.

9. The method as claimed in one of the preceding claims, **characterized in that** the fastening elements (10) are secured against rotation and fastened non-releasably to the orthosis or prosthesis components.

10. The method as claimed in one of the preceding claims, **characterized in that** the fastening elements (10) are completely covered by the base layer (2) on the side of the orthosis component or prosthesis component facing toward the body part.

11. The method as claimed in one of the preceding claims, **characterized in that** binding surfaces (14), which are positioned on the fastening elements (10) spaced apart from the base (11), of all the fastening elements (10) are pre-positioned in a plane or in a region between two parallel planes, and a spacing present between the bases (11) and the base layer (2) is filled via a compensating element or a compensating compound (7).

12. The method as claimed in one of the preceding claims, **characterized in that** the fastening elements (10) are pre-positioned in predetermined positions relative to each other and to a joint axis (3) of a joint of the body part and are arranged on or assigned to the base layer (2).

13. The method as claimed in claim 12, **characterized in that** the fastening elements (10) are arranged on a holder (110, 120) or a positioning device (100) and are pre-positioned relative to each other before they are arranged on or assigned to the base layer (2).

14. The method as claimed in one of the preceding claims, **characterized in that**, in order to form an orthosis with a distal component (22) and a proximal component (21), the at least one fiber composite material layer (8) is arranged on the support (1), in a region corresponding to the one in which a natural joint is located, and is cured in order to form a main body (20), wherein the fastening elements (10), before the curing, are arranged proximally and distally from a joint axis (3) and are embedded in the at least one fiber composite material layer (8), and **in that**, after the curing, the at least one fiber composite material layer (8), or a region of the base layer (2) of the main body (20) not covered by the fiber composite material layer (8), is separated in the region of the joint axis (3), in order to form from the main body (20) the distal component (22) and the proximal component (21) of the orthosis.

15. A method for the production of an orthosis, in which the orthosis components (21, 22) have been produced by the method as claimed in claim 14, and for securing a joint device (30) to the fastening elements (10).

16. An orthosis or prosthesis component for receiving or for fastening to a body part, wherein the orthosis or prosthesis component (21, 22) has a base layer (2) which can be arranged on a support (1) shaped in a manner corresponding to the shape of the body part, at least one fastening element (10) with a base (11) and with a form-fit element (12) protruding from the base (11) is arranged on the base layer (2), wherein the base (11) bears on or faces toward the base layer (2), and the fastening element (10) is embedded in at least one layer of fiber composite material (8), and the form-fit element (12) remains accessible from the side facing away from the base layer (2) after the at least one fiber composite material layer (8) has been cured, cwherein a plurality of fastening elements (10) are embedded which have a shaft (13) with or as a form-fit element (12) and at least one non-embedded binding surface (14), wherein the shafts (13) are oriented parallel to each other, **characterized in that** the fastening elements (10) are embedded non-releasably or in a manner secure against rotation in the at least one layer (8) of fiber composite material.

17. The orthosis or prosthesis component as claimed in claim 16, **characterized in that** binding surfaces (14) are oriented parallel to each other.

18. The orthosis or prosthesis component as claimed in claim 16 or 17, **characterized in that** the binding surfaces (14) are arranged in a plane (E1, E2) or offset between two mutually parallel planes or arranged at an angle to each other.

19. The orthosis or prosthesis component as claimed in one of claims 16 through 18, **characterized in that** the base layer (2) is formed from a fiber composite material land forms a closed surface on the side facing toward the body part.

20. The orthosis or prosthesis component as claimed in one of claims 16 through 19, **characterized in that** the fastening element (10) has a non-round base (11) and/or projections and/or recesses (16) for the securing against rotation.

21. The orthosis or prosthesis component as claimed in one of claims 16 through 20, **characterized in that** a thread is arranged or formed on or in the form-fit element (12).

22. The orthosis or prosthesis component as claimed in one of claims 16 through 21, **characterized in that** fastening devices (40) are arranged thereon for securing to a body part.

23. An orthosis composed of a plurality of orthosis components as claimed in one of claims 16 through 22, and of at least one joint device (30) secured to the fastening elements (10).

24. The orthosis composed of a plurality of orthosis components as claimed in one of claims 16 through 22, which are connected to each other to span a natural joint and have at least one predetermined separation point (6) or a predetermined separation region in which the joint axis (3) of the natural joint lies.

## Revendications

1. Procédé de fabrication de composants d'orthèse ou de prothèse pour la réception de ou la fixation à une partie du corps, comprenant les étapes consistant à :
a. appliquer une couche de base (2) sur un support (1) formé de manière à correspondre à la forme de la partie du corps,
b. disposer plusieurs éléments de fixation (10), présentant une base (11) et un élément de coopération de forme (12) dépassant de la base (11), sur la couche de base (2) dans des positions définies les uns par rapport aux autres, la base (11) des éléments de fixation (10) reposant sur la couche de base (2) ou étant tournée vers la couche de base (2),
c. poser au moins une couche (8) d'un matériau composite fibreux sur la couche de base (2) et incorporer la base (11), l'élément de coopération de forme (12) restant accessible depuis le côté détourné de la couche de base (2), et
d. faire durcir ladite au moins une couche de matériau composite fibreux (8),
**caractérisé en ce que**
après le durcissement, les éléments de fixation (10) sont incorporés dans ladite au moins une couche de matériau composite fibreux (8) de manière bloquée en rotation ou inamovible.

2. Procédé selon la revendication 1,
**caractérisé en ce que** la couche de base (2) est reliée à ladite au moins une couche de matériau composite fibreux (8), et la base (11) des éléments de fixation (10) est intégrée par stratification entre la couche de base (2) et ladite au moins une couche de matériau composite fibreux (8).

3. Procédé selon la revendication 1 ou 2,
**caractérisé en ce que** l'élément de coopération de forme (12) est réalisé ou disposé sur ou dans une emboîture (13), et l'emboîture (13) est incorporée partiellement dans ladite au moins une couche de matériau composite fibreux (8) et dépasse partiellement hors de la couche de matériau composite fibreux (8).

4. Procédé selon la revendication 3,
**caractérisé en ce que** les emboîtures (13) de plusieurs éléments de fixation (10), en particulier de tous les éléments de fixation (10), sont alignées parallèlement les unes aux autres.

5. Procédé selon l'une des revendications précédentes,
**caractérisé en ce qu'**au moins une surface d'attache (14) est disposée ou réalisée sur les éléments de fixation (10), laquelle est espacée de la base (11) et n'est pas recouverte par ladite au moins une couche de matériau composite fibreux (8).

6. Procédé selon l'une des revendications précédentes,
**caractérisé en ce que** la base (11) est fixée sur la couche de base (2).

7. Procédé selon l'une des revendications précédentes,
**caractérisé en ce que** le support (1) est réalisé sous la forme d'un modèle positif de la partie du corps, et le durcissement de ladite au moins une couche de matériau composite fibreux (8) s'effectue sur le support (1).

8. Procédé selon l'une des revendications précédentes,
**caractérisé en ce que** des évidements sont formés dans ladite au moins une couche de matériau composite fibreux (8) posée, à travers lesquels sont menées des parties des éléments de fixation (10).

9. Procédé selon l'une des revendications précédentes,
**caractérisé en ce que** les éléments de fixation (10) sont immobilisés sur les composants d'orthèse ou de prothèse de manière bloquée en rotation et inamovible.

10. Procédé selon l'une des revendications précédentes,
**caractérisé en ce que** les éléments de fixation (10) sont complètement recouverts par la couche de base (2) sur le côté du composant d'orthèse ou de prothèse qui est tourné vers la partie du corps.

11. Procédé selon l'une des revendications précédentes,
**caractérisé en ce que** des surfaces d'attache (14), positionnées sur les éléments de fixation (10) à distance de la base (11), de tous les éléments de fixation (10) sont pré-positionnées dans un plan ou dans une zone entre deux plans parallèles, et un interstice existant entre les bases (11) et la couche de base (2) est comblé par un élément de compensation ou une pâte de compensation (7).

12. Procédé selon l'une des revendications précédentes,
**caractérisé en ce que** les éléments de fixation (10) sont pré-positionnés dans des positions prédéterminées les uns par rapport aux autres et par rapport à un axe d'articulation (3) d'une articulation de la partie du corps et sont disposés sur la couche de base (2) ou associés à celle-ci.

13. Procédé selon la revendication 12,
**caractérisé en ce que** les éléments de fixation (10) sont disposés sur un élément de maintien (110, 120) ou sur un dispositif de positionnement (100) et sont pré-positionnés les uns par rapport aux autres avant d'être disposés sur la couche de base (2) ou associés à celle-ci.

14. Procédé selon l'une des revendications précédentes,
**caractérisé en ce que**, pour réaliser une orthèse avec un composant distal (22) et un composant proximal (21), ladite au moins une couche de matériau composite fibreux (8) est disposée sur le support (1), dans une zone qui correspond à celle dans laquelle se trouve une articulation naturelle, et est durcie pour former un corps de base (20), les éléments de fixation (10) étant disposés, avant le durcissement, de manière proximale et distale par rapport à un axe d'articulation (3) et étant incorporés dans ladite au moins une couche de matériau composite fibreux (8), et
**en ce qu'**après le durcissement, ladite au moins une couche de matériau composite fibreux (8) ou une zone de la couche de base (2) du corps de base (20) non recouverte par la couche de matériau composite fibreux (8) est coupée dans la zone de l'axe d'articulation (3), afin de former le composant distal (22) et le composant proximal (21) de l'orthèse à partir du corps de base (20).

15. Procédé pour fabriquer une orthèse, dans lequel les composants d'orthèse (21, 22) ont été fabriqués par le procédé selon la revendication 14, et pour immobiliser un dispositif d'articulation (30) sur les éléments de fixation (10).

16. Composant d'orthèse ou de prothèse pour la réception de ou la fixation à une partie du corps, dans lequel le composant d'orthèse ou de prothèse (21, 22) présente une couche de base (2) qui peut être disposée sur un support (1) formé de manière à correspondre à la forme de la partie du corps, au moins un élément de fixation (10), présentant une base (11) et un élément de coopération de forme (12) dépassant de la base (11), est disposé sur la couche de base (2), la base (11) reposant sur la couche de base (2) ou étant tournée vers celle-ci, et l'élément de fixation (10) est incorporé dans au moins une couche de matériau composite fibreux (8), et, après le durcissement de ladite au moins une couche de matériau composite fibreux (8), l'élément de coopération de forme (12) reste accessible depuis le côté détourné de la couche de base (2),
dans lequel
plusieurs éléments de fixation (10) sont incorporés, qui présentent une emboîture (13), comprenant ou faisant office d'élément de coopération de forme (12), et au moins une surface d'attache (14) non incorporée,
les emboîtures (13) sont alignées parallèlement les unes aux autres,
**caractérisé en ce que** les éléments de fixation (10) sont incorporés dans ladite au moins une couche de matériau composite fibreux (8) de manière bloquée en rotation ou inamovible.

17. Composant d'orthèse ou de prothèse selon la revendication 16, **caractérisé en ce que** les surfaces d'attache (14) sont alignées parallèlement les unes aux autres.

18. Composant d'orthèse ou de prothèse selon la revendication 16 ou 17, **caractérisé en ce que** les surfaces d'attache (14) sont disposées dans un plan (E1, E2) ou sont décalées entre deux plans parallèles l'un à l'autre ou sont disposées selon un angle l'une par rapport à l'autre.

19. Composant d'orthèse ou de prothèse selon l'une des revendications 16 à 18,
**caractérisé en ce que** la couche de base (2) est réalisée en un matériau composite fibreux et forme une surface fermée sur le côté tourné vers la partie du corps.

20. Composant d'orthèse ou de prothèse selon l'une des revendications 16 à 19,
**caractérisé en ce que** les éléments de fixation (10) présentent une base non ronde (11) et/ou des saillies et/ou des évidements (16) pour le blocage en rotation.

21. Composant d'orthèse ou de prothèse selon l'une des revendications 16 à 20,
**caractérisé en ce qu'**un pas de vis est disposé ou réalisé sur ou dans l'élément de coopération de forme (12).

22. Composant d'orthèse ou de prothèse selon l'une des revendications 16 à 21,
**caractérisé en ce que** des dispositifs de fixation (40) y sont disposés pour l'immobilisation sur une partie du corps.

23. Orthèse constituée de plusieurs composants d'orthèse selon l'une des revendications 16 à 22 et d'au moins un dispositif d'articulation (30) immobilisé sur les éléments de fixation (10).

24. Orthèse constituée de plusieurs composants d'orthèse selon l'une des revendications 16 à 22, qui sont reliés entre eux de manière à coiffer une articulation naturelle et qui présentent au moins un emplacement de séparation de consigne (6) ou une zone de séparation de consigne, dans lequel ou dans laquelle se trouve l'axe d'articulation (3) de l'articulation naturelle.
